# EUROPÄISCHE PATENTSCHRIFT

(11) **EP 2 398 386 B1**
(45) Veröffentlichungstag und Bekanntmachung des Hinweises auf die Patenterteilung: **09.08.2017**
(21) Anmeldenummer: 10705101.3
(22) Anmeldetag: 12.02.2010
(51) Int. Cl.: A61B 5/151

(54) **VERFAHREN ZUR HERSTELLUNG EINES ANALYTISCHEN MAGAZINS**
METHOD FOR PRODUCING AN ANALYTICAL MAGAZINE
PROCÉDÉ DE FABRICATION D'UN CHARGEUR ANALYTIQUE

(30) Priorität: 19.02.2009 EP 09153210; 20.08.2009 EP 09168336
(43) Veröffentlichungstag der Anmeldung: 28.12.2011
(73) Patentinhaber: Roche Diabetes Care GmbH, 68305 Mannheim (DE); F. Hoffmann-La Roche AG, 4070 Basel (CH)
(72) Erfinder: LIST, Hans, 64754 Hesseneck-Kailbach (DE); KRÄMER, Uwe, 68549 Ilvesheim (DE); LEICHNER, Wilhelm, 68307 Mannheim (DE)
(74) Vertreter: Herzog, Fiesser & Partner Patentanwälte PartG mbB
(86) Internationale Anmeldenummer: PCT/EP2010/000864
(87) Internationale Veröffentlichungsnummer: WO 2010/094426

(56) Entgegenhaltungen:
- EP-A- 2 042 098
- EP-A1- 1 360 932
- EP-A1- 1 997 429
- EP-A1- 2 050 392
- WO-A-03/070099
- WO-A-2005/104948
- WO-A-2006/031920
- US-A1- 2009 010 802

## Beschreibung

### Gebiet der Erfindung

Die vorliegende Erfindung betrifft ein Verfahren zur Herstellung eines analytischen Magazins, welches eingerichtet ist, um eine Mehrzahl von analytischen Hilfsmitteln aufzunehmen. Derartige analytische Magazine werden insbesondere in der medizinischen Diagnostik eingesetzt, um einen oder mehrere Analyten qualitativ oder quantitativ in Körperflüssigkeiten nachzuweisen. Beispielsweise kann es sich bei diesen Analyten um Metabolite, beispielsweise Blutglukose, handeln.

### Stand der Technik

Im Bereich der Diagnostik ist es in vielen Fällen notwendig, Proben von Körperflüssigkeit, insbesondere Blutproben oder Proben interstitieller Flüssigkeit, zu gewinnen, um darin Inhaltsstoffe, insbesondere bestimmte Analyte, nachweisen zu können. Beispiele derartiger Analyte sind Blutglukose, Gerinnungsparameter, Triglyceride, Lactat oder ähnliches. Entsprechend der nachgewiesenen Konzentrationen kann dann beispielsweise über eine entsprechende Behandlung entschieden werden.

In den genannten diagnostischen Verfahren werden in der Regel ein oder mehrere analytische Hilfsmittel eingesetzt, um die Proben an Körperflüssigkeit zu gewinnen und/oder zu analysieren. So können die analytischen Hilfsmittel beispielsweise Lanzetten umfassen, also Elemente, welche eingerichtet sind, um eine Öffnung in einer Haut eines Probanden zu erzeugen, durch welche die Körperflüssigkeit entnommen werden kann. Als Beispiel für derartige Lanzetten kann auf WO 01/36010 A1 verwiesen werden.

Weiterhin können die analytischen Hilfsmittel ein oder mehrere Testelemente mit Testchemien umfassen, welche eingerichtet sind, um bei Einwirkung des nachzuweisenden Analyten bestimmte nachweisbare Eigenschaften zu ändern. Beispielsweise können diese Analyten elektrochemisch nachweisbare Eigenschaften bzw. deren Änderungen umfassen und/oder optisch nachweisbare Eigenschaften. Auch für derartige Testchemien kann auf den Stand der Technik verwiesen werden, beispielsweise J. Hönes et al., Diabetes Technology & Therapeutics, Volume 10, Supplement 1, 2008, S-10 bis S-26.

Daneben sind auch integrierte Testelemente bekannt, welche sowohl dem Zweck der Erzeugung der Probe der Körperflüssigkeit als auch dem Zweck des Transports der Probe und gegebenenfalls sogar dem Zweck der qualitativen und/oder quantitativen Analyse dieser Probe dienen. Beispiele derartiger analytischer Hilfsmittel sind so genannte Microsampler, bei denen mittels einer Lanzette ein Einstich oder Einschnitt erzeugt wird, die Probe aufgenommen wird und zu einem oder mehreren Testfeldern mit der Testchemie transportiert wird. Diese Testfelder können separat von der Lanzette angeordnet sein, können jedoch auch Bestandteil der Lanzette selbst sein. Derartige Systeme, welche beispielsweise in US 2004/0193202 A1, US 2008/0249435 A1 oder in WO03/009759 A1 beschrieben sind, sind aufgrund ihres hohen Integrationsgrades besonders nutzerfreundlich.

Eine technische Herausforderung bei der Bereitstellung analytischer Systeme und analytischer Hilfsmittel besteht jedoch darin, diese in großen Mengen unter geeigneten Bedingungen bereitzustellen. So muss die Bereitstellung in der Regel derart erfolgen, dass die analytischen Hilfsmittel unter sterilen Bedingungen gelagert werden, beispielsweise durch entsprechende Versiegelungen. Gleichzeitig dürfen die Versiegelungen jedoch die Qualität der analytischen Hilfsmittel nicht beeinträchtigen und deren Benutzung nicht erschweren. Zu diesem Zweck werden die analytischen Hilfsmittel in der Regel mittels entsprechender Magazine, die im Folgenden auch als analytische Magazine bezeichnet werden, bereitgestellt. Für Systeme, die beispielsweise eine Kapillarblutanalyse vollautomatisch ohne Eingriffe des Probanden durchführen sollen, können beispielsweise in einem derartigen Magazin sowohl eine Vielzahl von Lanzetten, als auch eine Vielzahl von Testelementen, beispielsweise jeweils mit einer oder mehreren Testchemien, vorliegen.

Aus dem Stand der Technik ist eine Vielzahl unterschiedlicher analytischer Magazine bekannt. Grundsätzlich lassen sich, unabhängig von der Art des analytischen Hilfsmittels, drei Haupttypen von Magazinen unterscheiden, nämlich Rundmagazine (beispielsweise in Form von Trommeln und/oder Scheiben), Linearmagazine (beispielsweise in Form von Stapelmagazinen, Zick-Zack-Magazinen oder ähnlichem) und Bandmagazine, bei welchen die analytischen Hilfsmittel auf einem Band oder einer anderen Form von zumindest teilweise flexiblem Träger angeordnet sind. Diese Magazintypen lassen sich grundsätzlich auch im Rahmen der im Folgenden beschriebenen Erfindung einsetzen, bzw. modifizieren. Im Stand der Technik werden Rundmagazine beispielsweise in US 2006/0008389, US 2007/0292314, US 2006/0184064, US 2003/0212347 oder US 2002/0087056 beschrieben. Linearmagazine sind beispielsweise in US 6,036,924 oder in US 2003/0191415 beschrieben. Bandmagazine werden beispielsweise offenbart in US 2002/0188224, in US 2008/0103415, in EP 1360935 A1 oder in DE 19819407 A1.

US 2009/010802 A1 offenbart ein Verfahren zur Herstellung eines analytischen Magazins mit einer Mehrzahl von analytischen Hilfsmitteln in einer Mehrzahl von Kammern. Das Verfahren umfasst hierbei die Schritte: Bereitstellen mindestens eines ersten Bauteils des analytischen Magazins, wobei das erste Bauteil eine Mehrzahl von Aufnahmen umfasst; Bereitstellen einer Mehrzahl von analytischen Hilfsmitteln, wobei die analytischen Hilfsmittel wobei die analytischen Hilfsmittel durch mindestens ein Halteelement miteinander verbunden sind; Einbringen der analytischen Hilfsmittel in die Aufnahmen, wobei mehrere oder vorzugsweise alle Kammern bestückt werden; und Trennen der analytischen Hilfsmittel (134) von dem Halteelement.

EP 2 042 098 A offenbart ein Lanzettenmagazin mit mehreren Lanzetten zum Erzeugen einer Stichwunde zur Gewinnung einer Körperflüssigkeitsprobe, wobei das Lanzettenmagazin eine Mehrzahl von Lanzetten umfasst. Insbesondere lässt sich eine rationelle Fertigung dadurch erreichen, dass die Lanzetten und die Antriebsarme als Kranz gefertigt und in einem Arbeitsgang in eine Gehäusehälfte des Lanzettenmagazins eingesetzt werden. Der Lanzettenkreis kann durch einen Spritzgießprozess kostengünstig hergestellt werden. Die Lanzetten können einstückig mit den übrigen Elementen des Lanzettenkranzes aus einem Hartkunststoff hergestellt werden. Möglich ist es auch, die Lanzetten aus einem anderen Werkstoff, insbesondere Edelstahl, herzustellen und in Lanzettenhalterungen des Lanzettenkranzes einzusetzen. Vor dem Einsetzen des Lanzettenkranzes in eine Gehäusehälfte werden die Stege zwischen den Lanzettenhalterungen entfernt.

EP 2 050 392 A1 offenbart ein Lanzettenrad mit mehreren ringförmig angeordneten Lanzetten, die einen in einer Lanzettenspitze endenden Lanzettenkörper aufweisen. Das Lanzettenrad hat einen Träger, der die Lanzetten trägt, wobei die Lanzetten über Stege mit dem Träger verbunden sind, die eine Beweglichkeit der Lanzetten relatriv zu dem Träger ermöglichen.

Allgemein besteht der Nachteil bei analytischen Magazinen, insbesondere integrierten analytischen Magazinen mit kombinierten analytischen Hilfsmitteln mit Lanzettenfunktion und Testelementfunktion in einer Gewährleistung der Kontaminationsfreiheit und der Sterilität. Beispielsweise besteht eine Schwierigkeit darin, dass jedes Set aus Lanzette und Testelement von den jeweils anderen Sets getrennt aufbewahrt werden muss, da zumindest die Lanzetten bis unmittelbar vor deren Benutzung steril gehalten werden müssen.
Diese Anforderungen erhöhen jedoch wiederum den Aufwand für die Herstellung der analytischen Magazine. Aufgrund der unterschiedlichen Natur von Lanzetten und Testelementen ist deren Entstehungsgeschichte extrem unterschiedlich. Somit werden diese Elemente des Systems in der Regel in unterschiedlichen Darreichungsformen für die Montage des gesamten analytischen Magazins bereitgestellt. Diese Anforderungen bedingen, dass analytische Magazine in der Praxis in der Regel einzeln befüllt werden müssen. So müssen beispielsweise die Lanzetten und die Testelemente einzeln in die analytischen Magazine und/oder in einzelne Kammern der analytischen Magazine eingefügt werden, was in der Regel einen hohen apparativen Aufwand erfordert. Eine Ausnahme diesbezüglich bilden lediglich bandbasierte Systeme, bei welchen die einzelnen Elemente zunächst einzeln auf ein Trägerband aufgebracht werden können und dann aufgewickelt auf dieses Trägerband in ein Magazin eingefüllt werden können. In jedem Fall sind aber die einzelnen analytischen Hilfsmittel Stück für Stück zu applizieren, was einen erheblichen Herstellungsaufwand erfordert.

### Aufgabe der Erfindung

Es ist daher eine Aufgabe der vorliegenden Erfindung, ein Verfahren bereitzustellen, welches die Nachteile bekannter Verfahren zumindest weitgehend vermeidet. Insbesondere soll das vorgeschlagene Verfahren eine Herstellung von analytischen Magazinen ermöglichen, bei welcher der apparative Aufwand und somit der Gesamtaufwand für die Herstellung der analytischen Magazine erheblich verringert werden kann, ohne dass hierdurch die Qualität der analytischen Magazine und/oder der analytischen Hilfsmittel beeinträchtigt wird.

### Offenbarung der Erfindung

Diese Aufgabe wird durch das Verfahren mit den Merkmalen des Anspruchs 1 gelöst. Vorteilhafte Weiterbildungen der Erfindung, welche einzeln oder in Kombination realisiert werden können, sind in den abhängigen Ansprüchen dargestellt. Das analytische Magazin ist eingerichtet, um eine Mehrzahl von analytischen Hilfsmitteln in einer Mehrzahl von Kammern aufzunehmen. Unter einem analytischen Magazin ist somit eine Vorrichtung zu verstehen, welche als Einheit gehandhabt werden kann, welche beispielsweise ein gemeinsames Gehäuse aufweisen kann und welche allgemein in der Medizintechnik einsetzbar sein kann. Unter analytisch ist dabei allgemein die Möglichkeit eines Einsatzes für den qualitativen und/oder quantitativen Nachweis mindestens eines Analyten und/oder die Bestimmung mindestens einer weiteren messbaren Eigenschaft zu verstehen. Diesbezüglich kann beispielsweise auf die obige Beschreibung verwiesen werden. Insbesondere kann unter analytisch somit eine diagnostische Eigenschaft verstanden werden, also eine Verwendung für die Bestimmung mindestens einer Eigenschaft eines Körpers und/oder eines Bestandteils eines Körpers eines Probanden. Das analytische Magazin kann entsprechend in einem analytischen System eingesetzt werden, welches somit zu einem erfindungsgemäßen analytischen System wird. Beispielsweise kann es sich bei einem derartigen System um ein Messgerät handeln, mittels dessen mindestens ein Analyt, beispielsweise mindestens ein Metabolit, in einer Körperflüssigkeit des Probanden qualitativ und/oder quantitativ nachgewiesen wird. Beispielsweise kann es sich bei diesen Systemen um Blutglukosemessgeräte handeln.
Unter analytischen Hilfsmitteln sind allgemein im Rahmen der vorliegenden Erfindung Hilfsmittel zu verstehen, welche bei den oben beschriebenen analytischen Funktionen unterstützend eingesetzt werden können. Insbesondere kann es sich bei den analytischen Hilfsmitteln um medizinische und/oder diagnostische Hilfsmittel handeln, insbesondere um Hilfsmittel, welche eingerichtet sind, um bei einem qualitativen und/oder quantitativen Nachweis mindestens eines Analyten, beispielsweise eines oder mehrerer der oben genannten Analyten, in einer Köperflüssigkeit eines Probanden, beispielsweise Blut, interstitieller Flüssigkeit, Urin oder ähnlichen Körperflüssigkeiten, eingesetzt zu werden. Insbesondere können die analytischen Hilfsmittel als Einweg-Hilfsmittel (Disposables) ausgestaltet sein, also für den einmaligen Gebrauch bestimmt sein. Die analytischen Hilfsmittel können dementsprechend beispielsweise mindestens eine Lanzette umfassen, also ein Element, welches eingerichtet ist, um mindestens eine Öffnung in einer Haut des Probanden zu erzeugen, beispielsweise einem Ohrläppchen, einer Fingerbeere oder einem Unterarm des Probanden. Beispielsweise können diese Lanzetten ein oder mehrere Stechelemente umfassen, mit Nadelspitzen und/oder angeschliffenen Spitzen. Auch andere scharfkantige Elemente können alternativ oder zusätzlich verwendet werden, beispielsweise Klingen, scharfkantige Spitzen oder ähnliches. Die Lanzetten können beispielsweise aus stabförmigen Ausgangsmaterialien hergestellt werden, beispielsweise in Form von nadelförmigen Lanzetten. Besonders bevorzugt im Rahmen der vorliegenden Erfindung ist jedoch die Verwendung einer oder mehrerer Lanzetten, welche aus plattenförmigen Materialien, insbesondere Metallblechen, hergestellt sind. Dies wird unten noch näher erläutert.

Alternativ oder zusätzlich zu Lanzetten können die analytischen Hilfsmittel auch jeweils ein oder mehrere Testelemente umfassen. Diese Testelemente weisen mindestens eine Testchemie auf, welche eingerichtet ist, um bei Anwesenheit mindestens eines nachzuweisenden Analyten mindestens eine messbare Eigenschaft zu ändern. Diese Testchemie, welche eingerichtet ist, um alleine oder in Zusammenwirkung mit dem Analyten und/oder weiteren Hilfsstoffen die Anwesenheit oder - was hiervon umfasst sein soll - die Abwesenheit des mindestens einen Analyten zu indizieren, kann auf verschiedene Weisen ausgestaltet sein. Diesbezüglich kann beispielsweise wiederum auf den oben zitierten Artikel von J. Hönes et al. verwiesen werden. Weiterhin kann beispielsweise auf WO 2007/012494 A1 verwiesen werden, in welcher besonders feuchtigkeitsstabile Testchemien beschrieben werden. Die in diesen Druckschriften genannten Testchemien können, einzeln oder in Kombination, auch im Rahmen der vorliegenden Erfindung eingesetzt werden. Insbesondere können stark spezifische Testchemien eingesetzt werden, bei welchen der Nachweis spezifisch auf den mindestens einen Analyten reagiert. Die mindestens eine messbare Eigenschaft, aus deren Messung sich qualitativ oder quantitativ der mindestens eine Analyt nachweisen lässt, kann beispielsweise mindestens eine elektrochemische Eigenschaft und/oder mindestens eine optische Eigenschaft umfassen. Die Testchemie kann beispielsweise, wie unten näher ausgeführt wird, in Form von einem oder mehreren Testfeldern ausgeführt sein.

Weiterhin lassen sich die analytischen Hilfsmittel auch derart ausgestalten, dass diese als kombinierte Testelemente ausgestaltet sind. So können beispielsweise kombinierte Testelemente mit mindestens einer Lanzette und mindestens einer Testchemie verwendet werden, wobei die Testchemie eingerichtet sein kann, um bei Anwesenheit des mindestens einen nachzuweisenden Analyten mindestens eine messbare Eigenschaft zu ändern. Beispielsweise kann das Testelement unmittelbar in die Lanzette integriert sein. So kann beispielsweise die Testchemie am Ende der Lanzette aufgenommen sein und/oder Teile der Lanzette zu bedecken. Alternativ oder zusätzlich ist es jedoch auch möglich, dass die Lanzette und das Testelement getrennt ausgebildet sind, beispielsweise jeweils mindestens eine Lanzette und jeweils mindestens ein Testelement pro Kammer des analytischen Magazins. Diese Teile des analytischen Hilfsmittels können beispielsweise auch getrennt handhabbar sein, so dass beispielsweise die Lanzette von einem Aktor eines Systems gehandhabt werden kann, um eine Stechbewegung und/oder eine Sammelbewegung auszuführen, während das Testelement beispielsweise unverändert verbleibt, beispielsweise innerhalb der Kammer. So kann beispielsweise das System eingerichtet sein, um eine Stech- und/oder Sammelbewegung mittels der mindestens einen Lanzette und/oder einem in der Lanzette optional enthaltenen Kapillarelement durchzuführen, so dass während eines Stechvorgangs und/oder während einer Probennahmebewegung direkt Körperflüssigkeit von der Lanzette aufgenommen werden kann. Dabei kann zunächst ein Einstich in die Haut des Probanden erzeugt werden, Körperflüssigkeit gesammelt werden und diese dann, beispielsweise bei einer Rückwärtsbewegung der Lanzette, zurück in die Kammer hinein, auf das Testelement übertragen werden. Auch andere Ausgestaltungen sind möglich.

Alternativ oder zusätzlich zu Lanzetten und/oder Testelementen können die analytischen Hilfsmittel weitere Elemente umfassen, welche einem Analysezweck dienen. So können beispielsweise Transferelemente und/oder Sammelelemente umfasst sein, welche dem Zweck einer Aufnahme und/oder eines Transports von Körperflüssigkeit dienen. Beispielsweise kann mittels derartiger Transportelemente und/oder Sammelelemente eine Aufnahme von Blut und/oder interstitieller Flüssigkeit von einer Haut des Probanden und/oder einer Stelle innerhalb des Körpers des Probanden und/oder einer Stelle auf der Haut des Probanden erfolgen und/oder ein Transport zu einem Testelement, insbesondere einem oder mehreren Testfeldern. Ein derartiger Transport kann beispielsweise durch eine Transportbewegung erfolgen, mittels eines oder mehrerer Transportelemente, welche beweglich ausgestaltet sind und eine Menge der Probe der Körperflüssigkeit aufnehmen und transferieren können. Alternativ oder zusätzlich können auch andere Transportelemente und/oder Sammelelemente vorgesehen sein, beispielsweise Kapillaren und/oder Elemente mit Kapillarwirkung. Beispielsweise kann es sich dabei um geschlossene Kapillaren oder um Kapillarkanäle, insbesondere Kapillarspalte, handeln. Kombinierte analytische Hilfsmittel, welche mindestens eine Lanzettenfunktion und mindestens eine Kapillarfunktion aufweisen, werden im Folgenden auch als Microsampler bezeichnet.

Wie oben dargestellt, ist es besonders bevorzugt, wenn die analytischen Hilfsmittel derart in den Kammern aufgenommen sind, dass in einer Kammer genau ein analytisches Hilfsmittel aufgenommen ist. Umfasst das analytische Hilfsmittel selbst jeweils eine Mehrzahl analytischer Teil-Hilfsmittel, wie beispielsweise jeweils mindestens eine Lanzette und jeweils mindestens ein Testelement, so können beispielsweise das jeweils mindestens eine Testelement und/oder die jeweils mindestens eine Lanzette, welche für einen einzigen, gemeinsamen Test vorgesehen sind (beispielsweise eine einzige Aufnahme von Körperflüssigkeit und/oder Analyse von Körperflüssigkeit) in einer gemeinsamen Kammer aufgenommen sein. Diese Ausgestaltung, bei welcher in einer Kammer jeweils ein analytisches Hilfsmittel aufgenommen ist, beispielsweise mit jeweils mindestens einem Teil-Hilfsmittel in Form eines Testelements und/oder mit jeweils mindestens einem Teil-Hilfsmittel in Form einer Lanzette, ist insbesondere bei einem scheibenförmigen Magazin realisierbar oder auch bei anderen Gestaltungen von Magazinen, wie beispielsweise stangenförmigen Magazinen. Alternativ zu einer Ausgestaltung, bei welcher jedes analytische Hilfsmittel in einer separaten Kammer aufgenommen ist, ist auch eine Ausgestaltung möglich, bei welcher mehrere analytische Hilfsmittel gleicher Art oder verschiedener Art in einer Kammer aufgenommen sind. Ein Beispiel einer derartigen Ausgestaltung ist ein Bandmagazin, bei welchem ein Gutwickel mit einer Mehrzahl unbenutzter analytischer Hilfsmittel in einer ersten Kammer aufgenommen ist und ein Schlechtwickel mit einer Mehrzahl benutzter analytischer Hilfsmittel in einer zweiten Kammer. Auch andere Ausgestaltungen sind möglich.

Unter einer Kammer ist dabei allgemein ein Element zu verstehen, welches mindestens einen zumindest teilweise geschlossenen Hohlraum aufweist, in welchem das analytische Hilfsmittel aufgenommen sein kann. Der Hohlraum kann dabei auch eine oder mehrere Öffnungen umfassen, wie im Folgenden noch näher ausgeführt wird. Die Kammern können auch jeweils eine oder mehrere Teil-Kammern umfassen und können jeweils eine oder mehrere Kammerwände umfassen, welche einem Innenraum der Kammern zuweisen.

Das Verfahren zur Herstellung des analytischen Magazins umfasst die im Folgenden beschriebenen Verfahrensschritte. Die Verfahrensschritte werden vorzugsweise, jedoch nicht notwendigerweise, in der dargestellten Reihenfolge durchgeführt. Auch andere Reihenfolgen sind grundsätzlich möglich. Weiterhin kann das Verfahren zusätzliche, nicht beschriebene Verfahrensschritte umfassen. Weiterhin können einzelne oder mehrere Verfahrensschritte wiederholt durchgeführt werden und/oder können zeitlich parallel und/oder zeitlich überlappend durchgeführt werden.

In einem ersten Verfahrensschritt wird mindestens ein erstes Bauteil des analytischen Magazins bereitgestellt. Dieses erste Bauteil kann beispielsweise ein Bauteil eines Gehäuses des analytischen Magazins umfassen. Beispielsweise kann das Bauteil zumindest teilweise steif ausgestaltet sein. Das Bereitstellen kann beispielsweise manuell und/oder auch automatisch erfolgen. Das erste Bauteil weist dabei eine Mehrzahl von Aufnahmen auf Beispielsweise kann für jede der oben genannten Kammern jeweils mindestens eine Aufnahme vorgesehen sein, welche der jeweiligen Kammer zugeordnet ist. Diese Aufnahmen können beispielsweise Vertiefungen, Schienen, Rillen, Nuten, Stege, Wände, Vorsprünge oder ähnliche Elemente umfassen, welche in der Lage sind, die analytischen Hilfsmittel und/oder Teile dieser analytischen Hilfsmittel zumindest teilweise zu fixieren und/oder innerhalb vorgegebener Grenzen eine Änderung der Lage und/oder Ausrichtung dieser analytischen Hilfsmittel zu verhindern. Die Aufnahmen können auch Bestandteil der späteren Kammern sein, beispielsweise in Form von Teil-Kammern, beispielsweise nach oben geöffnete Teil-Kammern oder Kammern, welche beispielsweise in einem späteren Verfahrensstadium verschlossen werden, insbesondere durch ein weiteres Bauteil, beispielsweise durch einen Deckel. Beispielsweise können die Aufnahmen Vertiefungen umfassen, welche später einen Teil der Wände der Kammern darstellen.

In einem weiteren Verfahrensschritt wird eine Mehrzahl analytischer Hilfsmittel der oben beschriebenen Art bereitgestellt. Bestehen die jeweils in einer Kammer aufgenommenen analytischen Hilfsmittel jeweils aus verschiedenen Arten analytischer Teil-Hilfsmittel, so können diese Teil-Hilfsmittel jeweils in unterschiedlichen Verfahrensschritten bereitgestellt werden und/oder auch mit allen oder einigen weiteren Teil-Hilfsmitteln gemeinsam bereitgestellt werden. Das Bereitstellen kann wiederum beispielsweise manuell oder auch ganz oder teilweise automatisiert erfolgen.

Im Unterschied zum Stand der Technik wird jedoch bei dem vorgeschlagenen Herstellungsverfahren gemäß einem ersten Aspekt der vorliegenden Erfindung vorgeschlagen, die analytischen Hilfsmittel nicht Stück für Stück bereitzustellen und in das Magazin einzufügen, sondern vorzugsweise alle auf einmal in das Magazin einzufügen. Dabei können alle Kammern gleichzeitig mit den analytischen Hilfsmitteln und/oder, was gleichbedeutend sein soll, mit Teil-Hilfsmitteln bestückt werden, wenn die analytischen Hilfsmittel jeweils für sich genommen aus mehreren Teil-Hilfsmitteln zusammengesetzt sind. Zumindest sollten mehrere Kammern mit mindestens einer Art von analytischem Hilfsmittel und/oder Teil-Hilfsmittel gleichzeitig bestückt werden, vorzugsweise alle Kammern. Hierdurch lässt sich der Montageaufwand erheblich reduzieren.

Dementsprechend wird vorgeschlagen, die bereitgestellten analytischen Hilfsmittel (oder, gleichbedeutend, die Teil-Hilfsmittel) durch mindestens ein Halteelement miteinander zu verbinden und vorzugsweise zueinander auszurichten. Die Bereitstellung kann dann in dieser verbundenen und vorzugsweise ausgerichteten Form erfolgen. Unter einem Halteelement ist dabei allgemein ein Element zu verstehen, welches für die gemeinsame Bereitstellung der Mehrzahl der analytischen Hilfsmittel geeignet ist. Beispiele dieses Halteelements werden unten näher beschrieben. Unter einer Ausrichtung der analytischen Hilfsmittel zueinander kann dabei beispielsweise eine zumindest weitgehende Fixierung einer absoluten Position und/oder einer räumlichen Ausrichtung (beispielsweise einer Winkelausrichtung) der analytischen Hilfsmittel zueinander verstanden werden. Hierbei sind jedoch auch leichte Abweichungen möglich, welche im Rahmen beispielsweise vorgegebener Toleranzen liegen können, welche beispielsweise durch die Toleranzen der Aufnahme der analytischen Hilfsmittel in den Kammern vorgegeben sein können.

In einem weiteren Verfahrensschritt werden die analytischen Hilfsmittel oder, was im Rahmen der vorliegenden Erfindung gleichbedeutend sein soll, die Teil-Hilfsmittel, in die Aufnahmen eingebracht. Dieses Einbringen kann beispielsweise durch einfaches Einlegen, Einstecken oder ähnliches erfolgen und kann beispielsweise wiederum manuell oder auch zumindest teilweise automatisiert erfolgen. Das Einbringen erfolgt, entsprechend der Bereitstellung der Mehrzahl der analytischen Hilfsmittel, dabei für eine Mehrzahl oder vorzugsweise alle Kammern zumindest im Wesentlichen gleichzeitig, also im Wesentlichen in einem Verfahrensschritt für alle im vorhergehend beschriebenen Schritt bereitgestellten analytischen Hilfsmittel bzw. Teil-Hilfsmittel.

In einem weiteren Verfahrensschritt werden dann die analytischen Hilfsmittel von dem Halteelement getrennt. Das Trennen kann vorzugsweise ganz oder teilweise nach und/oder bei dem Einbringen der Teil-Hilfsmittel in die Aufnahmen erfolgen. Unter einem Trennen beim Einbringen ist dabei ein Trennen bei einem oder mehreren Verfahrensschritte zu verstehen, welche notwendig sind, um die Teil-Hilfsmittel in die Aufnahme einbringen zu können. Die Herstellung der Teilhilfsmittel kann zu diesem Zeitpunkt im Wesentlichen abgeschlossen sein, so dass diese zu diesem Zeitpunkt noch verbunden sein können. Die Trennung kann dann unmittelbar vor und/oder während dieser Verfahrensschritte erfolgen und/oder zu einem Zeitpunkt, zu dem die Teil-Hilfsmittel bereits teilweise in die Aufnahme eingebracht sind und/oder zu einem Zeitpunkt, zu dem die Teil-Hilfsmittel bereits vollständig in die Aufnahme eingebracht sind. Alternativ oder zusätzlich kann jedoch auch ein vollständiges oder teilweises Trennen während und/oder vor dem Einbringen in die Aufnahmen erfolgen. In diesem Fall kann beispielsweise eine Fixierungsvorrichtung verwendet werden, um die analytischen Hilfsmittel und/oder Teil-Hilfsmittel vorübergehend nach dem Trennen zu fixieren, bevor diese in die Aufnahmen eingebracht werden. Das Trennen kann beispielsweise durch übliche Trennverfahren erfolgen, welche insbesondere auch der Art und Weise, in der die analytischen Hilfsmittel bzw. Teil-Hilfsmittel mit dem Halteelement verbunden sind, angepasst sein kann. Beispielsweise können für diese Trennung, wie unten noch näher ausgeführt wird, Bruchverfahren, Schneideverfahren (insbesondere Laserschneideverfahren und/oder mechanische Schneideverfahren), Stanzverfahren, chemische Trennverfahren oder Kombinationen der genannten und/oder anderer Trennverfahren eingesetzt werden.
Das vorgeschlagene Verfahren weist gegenüber bekannten Verfahren eine Vielzahl von Vorteilen auf Als hauptsächlicher Vorteil ist die starke Vereinfachung des Aufwandes der Herstellung zu nennen. So lassen sich mit geringstem Aufwand beispielsweise analytische Magazine herstellen, bei welchen vorzugsweise die analytischen Hilfsmittel in unterschiedlichen Kammern voneinander getrennt angeordnet sind. Die analytischen Hilfsmittel können beispielsweise ganz oder teilweise unabhängig voneinander, d.h. unabhängig von den in den übrigen Kammern aufgenommenen analytischen Hilfsmitteln, handhabbar sein, im Gegensatz beispielsweise zu analytischen Hilfsmitteln, welche auf Bändern aufgenommen sind. Dennoch lässt sich der Aufwand für die Bestückung der einzelnen Kammern mit analytischen Hilfsmitteln durch das vorgeschlagene Verfahren erheblich reduzieren, da die einzelnen Kammern nunmehr nicht mehr einzeln bestückt werden müssen. So ist nunmehr eine Bestückung von Gruppen von Kammern und/oder sämtlicher Kammern gleichzeitig möglich. Diese Vorteile werden erzielt ohne Inkaufnahme von Nachteilen hinsichtlich Qualitätsverlusten, da die Sterilität der einzelnen Kammern beispielsweise durch entsprechende, unten noch näher beschriebene Versiegelungen gewährleistet sein kann.

Das vorgeschlagene Verfahren lässt sich auf verschiedene Weisen vorteilhaft weiterbilden. So ist das mindestens eine erste Bauteil des analytischen Magazins vorzugsweise als im Wesentlichen starres Bauteil ausgestaltet, also als Bauteil, welches zumindest unter Einwirkung seiner eigenen Gewichtskraft keinen wesentlichen Verbiegungen und/oder sonstigen Formänderungen unterworfen ist. Dementsprechend sind die oben beschriebenen Aufnahmen vorzugsweise in einer fest vorgegebenen Ausrichtung und/oder Orientierung zueinander angeordnet. Dementsprechend kann auch, wie oben dargestellt, das mindestens eine Halteelement vorzugsweise im Wesentlichen starr ausgestaltet sein.

Das analytische Magazin kann grundsätzlich die Mehrzahl von Kammern in einer beliebigen Anordnung zueinander umfassen. So sind beispielsweise Stangenmagazine, Reihenmagazine, Zickzack-Magazine oder ähnliches denkbar. Insbesondere kann auf die oben genannten Arten von Magazinen verwiesen werden. Besonders bevorzugt ist es, wenn das analytische Magazin eine Scheibenform aufweist, insbesondere eine Form einer Kreisscheibe und/oder einer Kreisringscheibe. Dementsprechend können die Kammern und/oder die Aufnahmen in dem scheibenförmigen analytischen Magazin im Wesentlichen in radialer Ausrichtung angeordnet sein. Beispielsweise kann das analytische Magazin in Form der Kreisscheibe und/oder Kreisringscheibe derart ausgestaltet sein, dass mittels der analytischen Hilfsmittel und/oder mittels mindestens eines der in jeder Kammer aufgenommenen analytischen Hilfsmittels und/oder mittels mindestens eines Teil-Hilfsmittels eine Probennahmebewegung durchgeführt werden kann. Unter einer Probennahmebewegung kann beispielsweise eine Stechbewegung und/oder eine Sammelbewegung zum Erzeugen und/oder Sammeln und/oder Transferieren einer Probe und/oder eines Teils einer Probe von Körperflüssigkeit verstanden werden. Entsprechend kann diese Probennahmebewegung beispielsweise in radialer Richtung erfolgen. Zu diesem Zweck kann beispielsweise bei der Kreisringscheibe im Inneren des Magazins mindestens eine Öffnung vorgesehen sein, in welche beispielsweise mindestens ein Aktor und/oder ein Teil eines Aktorsystems eines analytischen Systems eingreift, um an die analytischen Hilfsmittel und/oder Teil-Hilfsmittel in jeder Kammer (beispielsweise nacheinander) ankoppeln zu können und die Probennahmebewegung durchzuführen. Für die Ankopplung kann beispielsweise auf den oben zitierten Stand der Technik verwiesen werden, beispielsweise die WO 02/36010 A1. Auch andere Arten der Ankopplung sind jedoch grundsätzlich möglich. Ausführungsbeispiele von kreisscheibenförmigen und/oder kreisringscheibenförmigen Magazinen werden unten näher beschrieben.

Entsprechend kann auch das mindestens eine Halteelement auf die Ausgestaltung des Magazins angepasst sein. So kann beispielsweise bei einem stangenförmigen Magazin das Halteelement ausgestaltet sein, um die analytischen Hilfsmittel in paralleler Anordnung zueinander bereitzustellen. Bei einem kreisscheibenförmigen und/oder kreisringförmigen analytischen Magazin kann das Halteelement beispielsweise ausgestaltet sein, um die analytischen Hilfsmittel in einer radialen Ausrichtung zueinander bereitzustellen. Wie oben dargestellt, umfassen die analytischen Hilfsmittel Lanzetten und/oder Microsampler als analytische Hilfsmittel und/oder Teil-Hilfsmittel, welche beispielsweise mittels des Halteelements in einer radialen Ausrichtung zueinander, also einer strahlenförmigen Ausrichtung zueinander, beispielsweise in äquidistanter Anordnung, bereitgestellt werden können. Diese Bereitstellung kann beispielsweise derart erfolgen, dass die Spitzen dieser Lanzetten und/oder Microsampler jeweils radial nach außen weisen.
Das Halteelement kann grundsätzlich vergleichsweise komplex aufgebaut sein und kann beispielsweise eine Vielzahl von Teilelementen umfassen. Entsprechend kann das Halteelement für eine Mehrzahl von Bestückungsprozessen ausgestaltet sein. Besonders bevorzugt ist es jedoch, wenn das Halteelement als Einweg-Halteelement ausgestaltet ist, welches für genau einen Bestückungsprozess oder eine begrenzte Anzahl von Bestückungsprozessen ausgestaltet ist. Dementsprechend ist es besonders bevorzugt, das Halteelement vergleichsweise einfach auszugestalten, beispielsweise als einstückiges Halteelement. Die analytischen Hilfsmittel sind ganz oder teilweise aus einem Grundwerkstoff des Halteelements herausgearbeitet sein. Erfindungsgemäß ist dies ein metallischer Grundwerkstoff, aus welchem in einem oder mehreren Arbeitsschritten analytische Hilfsmittel in Form von Lanzetten und/oder Microsamplern herausgearbeitet werden, so dass das eigentliche Halteelement und die analytischen Hilfsmittel oder Teile derselben entstehen. Zum Herausarbeiten können dabei grundsätzlich beliebige, beispielsweise mechanische und/oder chemische Verfahren verwendet werden, beispielsweise Ätzverfahren und/oder Schneideverfahren und/oder Laserverfahren. Das Halteelement kann beispielsweise mindestens eine einfache Scheibe umfassen, also ein ebenes, im Wesentlichen plattenförmiges Element, dessen laterale Ausdehnungen seine Dicke um ein Vielfaches überschreiten. Beispielsweise kann das scheibenförmige Element eine einfache Metallscheibe umfassen. Beispielsweise kann diese Metallscheibe als im Wesentlichen rechteckige und/oder runde Metallscheibe ausgestaltet sein, was insbesondere bei der Ausgestaltung des analytischen Magazins als rundes analytisches Magazin, insbesondere mit einer radialen Ausrichtung der analytischen Hilfsmittel, bevorzugt ist. Beispielsweise können die analytischen Hilfsmittel ganz oder teilweise aus dieser Scheibe herausgearbeitet sein, so dass die analytischen Hilfsmittel gebildet werden und die verbleibende Scheibe das Halteelement oder einen Teil derselben bildet.

Die analytischen Hilfsmittel werden zumindest teilweise einstückig mit dem Halteelement hergestellt werden. Dies ist insbesondere dann bevorzugt, wenn das Halteelement als Einweg-Halteelement ausgestaltet ist. Die analytischen Hilfsmittel können dabei vollständig oder teilweise einstückig mit dem Halteelement ausgestaltet sein. Sind mehrere Teil-Hilfsmittel pro analytischem Hilfsmittel vorgesehen, so können eines, mehrere oder alle dieser Teil-Hilfsmittel einstückig mit dem Halteelement ausgestaltet sein.

Die einstückige Ausgestaltung kann beispielsweise dadurch erfolgen, dass die analytischen Hilfsmittel oder, was gleichbedeutend sein soll, Teil-Hilfsmittel, in einem oder mehreren Herstellschritten aus einem Roh-Element des Halteelements herausgearbeitet werden können. Dieses Roh-Element kann beispielsweise wiederum ein plattenförmiges Element, insbesondere ein scheibenförmiges Element, beispielsweise eine Metallscheibe, umfassen. Das Herausarbeiten der analytischen Hilfsmittel bzw. Teil-Hilfsmittel kann beispielsweise durch bekannte Arbeitsschritte erfolgen, insbesondere Ätzprozesse. So können insbesondere Lanzetten mit beispielsweise mittels eines oder mehrerer Ätzprozesse aus dem Roh-Element des Halteelements herausgearbeitet werden. Auch für andere analytische Hilfsmittel bzw. Teil-Hilfsmittel ist die Verwendung mindestens eines Ätzprozesses vorteilhaft. Alternativ oder zusätzlich zu Ätzprozessen können jedoch auch andere Arten von Herstellungsprozessen verwendet werden, insbesondere für die Herausarbeitung der analytischen Hilfsmittel bzw. Teil-Hilfsmittel aus den Roh-Elementen, beispielsweise Schneideprozesse, Stanzprozesse oder ähnliches.

Eine weitere, sehr rationelle Form von Halteelementen ist ein Halteelement in Form eines langen Streifens oder umfassend einen langen Streifen, der eine Vielzahl von Anordnungen von analytischen Hilfsmitteln und/oder Teilhilfsmitteln, beispielsweise Lanzetten und/oder Microsamplern, beinhaltet. Dies erlaubt beispielsweise die Bearbeitung von Rolle zu Rolle.

Wie oben dargestellt, kann das Trennen der analytischen Hilfsmittel bzw. Teil-Hilfsmittel von dem Halteelement unter Verwendung eines oder mehrerer entsprechender Trennverfahren erfolgen. Wie oben dargestellt, ist insbesondere ein Bruchverfahren bevorzugt. Für dieses Bruchverfahren, jedoch auch für andere Arten von Trennverfahren, ist es bevorzugt, wenn vor dem Trennen der analytischen Hilfsmittel von dem Halteelement zwischen dem analytischen Hilfsmittel und dem Halteelement mindestens eine Verbindung vorgesehen wird. Diese Verbindung kann auf die besondere Verwendung des mindestens einen Trennverfahrens angepasst sein. Beispielsweise kann diese Verbindung mindestens eine Brücke und/oder mindestens ein anderes Verbindungselement umfassen, welches vorzugsweise ebenfalls einstückig mit dem Halteelement und/oder den analytischen Hilfsmitteln ausgestaltet ist. Diese Verbindung kann vorzugsweise, insbesondere bei Verwendung mindestens eines Bruchverfahrens, mindestens eine Sollbruchstelle umfassen. Die Sollbruchstelle kann beispielsweise eine Verjüngung umfassen und/oder eine Ritzung und/oder eine andere Art der Schwächung einer Materialstärke des Verbindungselements. Auch eine gezielte Verminderung der Materialfestigkeit oder eine Versprödung im Bereich der Sollbruchstelle ist möglich, beispielsweise eine gezielte Erzeugung von Glashärte in einem ansonsten zähelastischen Stahl, beispielsweise mittels eines Lasers. Vorzugsweise ist die Verbindung derart ausgestaltet, dass nach deren Trennung bzw. nach dem Abtrennen der analytischen Hilfsmittel von dem Halteelement im Wesentlichen keine störenden Reste an dem analytischen Hilfsmittel, beispielsweise der Lanzette, verbleiben, welche später die Funktion des analytischen Hilfsmittels beeinträchtigen könnten. So kann beispielsweise mittels der genannten Verjüngungen und/oder Sollbruchstellen sichergestellt werden, dass ein sauberer Bruch entsteht, so dass beispielsweise ein Gleiten der Lanzetten und/oder anderer analytischer Hilfsmittel und/oder Teil-Hilfsmittel für eine Probennahmebewegung nicht beeinträchtigt wird. Insbesondere kann eine Sollbruchstelle derart ausgestaltet sein, dass diese von einer Kante des analytischen Hilfsmittels aus nach innen versetzt ist, beispielsweise in eine Taille des analytischen Hilfsmittels. Dies hat den Vorteil, dass gegebenenfalls beim Trennen des analytischen Hilfsmittels von dem Halteelement verbleibende Bruchreste ein Gleiten des analytischen Hilfsmittels in der Kammer nicht behindern.

Allgemein ist es bevorzugt, wenn die Kammern und/oder die analytischen Hilfsmittel derart ausgestaltet sind, dass die analytischen Hilfsmittel für eine Probennahmebewegung ganz oder teilweise beweglich gelagert sind. Dabei kann die bewegliche Lagerung für das analytische Hilfsmittel als Ganzes erfolgen oder auch für lediglich ein oder mehrere Teil-Hilfsmittel der analytischen Hilfsmittel, beispielsweise eine oder mehrere Lanzetten, während beispielsweise Testelemente fest innerhalb der Kammern und/oder an anderen Stellen des analytischen Magazins verbleiben können. Die bewegliche Lagerung kann beispielsweise derart erfolgen, dass die analytischen Hilfsmittel, wie unten noch näher ausgeführt wird, während einer Aufbewahrung der analytischen Hilfsmittel in den Kammern des Magazins vollständig oder teilweise fixiert sind, wohingegen für eine Probennahmebewegung diese Fixierung freigegeben und/oder überwunden wird. Die Probennahmebewegung kann, wie oben ausgeführt, beispielsweise durch ein mit dem analytischen Magazin zusammenwirkendes und/oder das analytische Magazin umfassendes analytisches System, beispielsweise ein Messgerät, erfolgen, welches beispielsweise über einen oder mehrere entsprechende Aktoren verfügen kann. Diese Aktoren können eingerichtet sein, um mit den analytischen Hilfsmitteln in den Kammern und/oder mit Teil-Hilfsmitteln dieser analytischen Hilfsmittel zusammenzuwirken und an diese anzukoppeln, vorzugsweise einzeln. Diese Aktoren, welche auch Teile des Magazins selbst umfassen können, können beispielsweise entsprechende Ankopplungselemente und/oder Probennahmeelemente umfassen, mittels derer die Ankopplungen und/oder Probennahmebewegungen durchgeführt werden können, beispielsweise einen oder mehrere Greifer, Haken, Stößel, Schieber oder Kombinationen der genannten und/oder anderer Elemente. Vorzugsweise können die Probennahmebewegung und/oder das System derart eingerichtet sein, dass die Probennahmebewegung eine Hinbewegung zur Haut des Probanden umfasst, ggf. einschließlich einer Einstichbewegung in die Haut des Probanden, gefolgt von einer Rückbewegung, weg von der Haut des Probanden. Beispielsweise kann die Rückbewegung eine Re-Magazinierung umfassen, also eine Bewegung, bei welcher das mindestens eine analytische Hilfsmittel bzw. Teil-Hilfsmittel wieder vollständig oder teilweise in der Kammer und/oder einer anderen Kammer des analytischen Magazins aufgenommen wird. Auf diese Weise lässt sich eine hygienisch einwandfreie Entsorgung der analytischen Hilfsmittel gewährleisten.

Wie oben dargestellt, kann das Verfahren weitere Verfahrensschritte umfassen. So kann beispielsweise das analytische Magazin neben dem ersten Bauteil weitere Bauteile umfassen. Besonders bevorzugt ist es jedoch, wenn außer den analytischen Hilfsmitteln und dem mindestens einem ersten Bauteil eine möglichst geringe Anzahl weiterer Bauteile vorgesehen ist, beispielsweise eine Anzahl von einem, zwei, drei oder vorzugsweise maximal vier weiteren Bauteilen. Auf diese Weise lässt sich eine möglichst einfache Herstellung des analytischen Magazins gewährleisten.

Das Verfahren kann insbesondere mindestens einen weiteren Verfahrensschritt umfassen, in welchem mindestens ein zweites Bauteil aufgebracht wird. Dieses zweite Bauteil kann beispielsweise wiederum ein Bauteil eines Gehäuses des analytischen Magazins sein. Das zweite Bauteil kann beispielsweise direkt oder indirekt auf das mindestens eine erste Bauteil aufgebracht werden. So kann das erste Bauteil beispielsweise, wie oben dargestellt, als Bodenteil eines Gehäuses ausgestaltet sein, wohingegen das zweite Bauteil beispielsweise als Deckelteil des Gehäuses ausgestaltet ist oder umgekehrt. Auch andere Ausgestaltungen sind möglich. Das zweite Bauteil kann beispielsweise unter Zwischenschaltung weiterer Bauteile auf das erste Bauteil aufgebracht werden. Das erste Bauteil und das zweite Bauteil können dabei durch einen oder mehrere Verbindungen miteinander verbunden werden, beispielsweise kraftschlüssige und/oder formschlüssige und/oder stoffschlüssige Verbindungen. Besonders bevorzugt sind stoffschlüssige Verbindungen, beispielsweise in Form von Klebeverbindungen und/oder Schweißverbindungen, insbesondere durch Laserschweißen und/oder Ultraschallschweißen.

Bei dem Schritt des Aufbringens des mindestens einen zweiten Bauteils, insbesondere auf das erste Bauteil, können beispielsweise die Kammern ausgebildet werden oder weitergebildet werden. Diese Kammern können beispielsweise dadurch entstehen, dass die oben genannten Aufnahmen in dem ersten Bauteil Teil-Wände der Kammern bilden, wohingegen Bestandteile des zweiten Bauteils weitere Teil-Wände der Kammern bilden. Auch das zweite Bauteil kann dementsprechend beispielsweise Vertiefungen und/oder ähnliche Bestandteile der Kammern umfassen, welche später Teil der Kammern bilden. Die durch das Aufbringen des mindestens einen zweiten Bauteils vorzugsweise gebildeten oder weitergebildeten Kammern können nach dem Aufbringen des zweiten Bauteils auch noch teilweise geöffnet vorliegen, beispielsweise mit einer oder mehreren unten noch im Detail beschriebenen Öffnungen. Insbesondere können bei dem Aufbringen des mindestens einen zweiten Bauteils die Aufnahmen des ersten Bauteils mit den darin aufgenommenen analytischen Hilfsmitteln bzw. Teil-Hilfsmitteln der analytischen Hilfsmittel zumindest weitgehend verschlossen werden. Unter einem zumindest weitgehenden Verschließen ist dabei ein Vorgang zu verstehen, bei welchem die räumlichen Begrenzungen der Kammern zumindest weitgehend definiert werden. Wie oben dargestellt, können dabei jedoch eine oder mehrere Öffnungen, insbesondere in den Kammerwänden, verbleiben. Beispielsweise kann mindestens eine Probennahmeöffnung vorgesehen sein, beispielsweise auf einer bei der Benutzung des analytischen Magazins bzw. des analytischen Systems dem Probanden zuweisenden Seite des Magazins, beispielsweise einer äußeren Umfangsseite eines kreisscheibenförmigen und/oder kreisringförmigen Magazins. Durch diese Probennahmeöffnungen, wobei beispielsweise mindestens eine derartige Probennahmeöffnung pro Kammer vorgesehen sein kann, können die analytischen Hilfsmittel und/oder Teil-Hilfsmittel aus den Kammern austreten, beispielsweise um die oben genannten Probenahmenbewegungen durchzuführen. Alternativ oder zusätzlich zu Probennahmeöffnungen können Aktoröffnungen vorgesehen sein, beispielsweise mindestens eine Aktoröffnung pro Kammer. Diese Aktoröffnungen können ausgestaltet sein, damit ein Aktor und/oder ein Teil eines Aktors, insbesondere eines Aktors des analytischen Systems, vollständig oder teilweise in die Kammern eindringen kann, um das mindestens eine analytische Hilfsmittel zu einer Probennahmebewegung anzuregen bzw. mittels dieses analytischen Hilfsmittels bzw. Teil-Hilfsmittels eine Probennahmebewegung durchzuführen. Diese Aktoröffnungen können beispielsweise auf einer den Probennahmeöffnungen gegenüberliegenden Seite vorgesehen sein, beispielsweise einer von der Hautoberfläche des Probanden abgewandten Seite des Magazins, beispielsweise einem Innenumfang einer Kreisringscheibe.

Alternativ oder zusätzlich können die Aktoröffnungen jedoch auch an Seitenflächen der Kammern vorgesehen sein, je nach Art der Ankopplung des Aktors an die analytischen Hilfsmittel.

Alternativ oder zusätzlich zu der mindestens einen Probennahmeöffnung und/oder der mindestens einen Aktoröffnung können weiterhin Messöffnungen vorgesehen sein, beispielsweise jeweils mindestens eine Messöffnung pro Kammer. Durch diese Messöffnungen können beispielsweise Messungen an den vollständig oder teilweise in den Kammern aufgenommenen optionalen Testelementen vorgenommen werden, beispielsweise optische und/oder elektrische Messungen. Beispielsweise können die Messöffnungen Messfenster umfassen, welche geöffnet oder auch mit einem transparenten Material verschlossen ausgestaltet sein können, um beispielsweise eine Änderung einer optischen Eigenschaft, beispielsweise eine Farbänderung, an einem oder mehreren Testfeldern messen zu können.

Alternativ oder zusätzlich zu den genannten Arten von Öffnungen können beispielsweise auch Testelementöffnungen vorgesehen sein, vorzugsweise wiederum beispielsweise mindestens eine Testelementöffnung pro Kammer. Durch diese Testelementöffnungen können ein oder mehrere Testelemente vollständig oder teilweise in die Kammern eingebracht werden. Wie unten näher beschrieben, kann dies beispielsweise derart erfolgen, dass ein oder mehrere Testelementfelder derart von außen auf diese Testelementöffnungen aufgebracht und/oder zumindest teilweise in diese Testelementöffnungen eingebracht werden, dass Teile oder Bereiche dieser Testfelder dem Innenraum der Kammern zuweisen. Diese Bereiche, welche somit im Inneren der Kammern aufgenommen sind, können damit für sich abgetrennte Teil-Hilfsmittel in Form von Testelementen bilden, welche jeweils einer Kammer zugeordnet sein können. Dies wird unten noch näher beschrieben.

Bei dem Aufbringen des mindestens einen zweiten Bauteils können insbesondere die analytischen Hilfsmittel in den Kammern, insbesondere den Aufnahmen des ersten Bauteils, zumindest weitgehend gegen eine unbeabsichtigte Lageänderung, insbesondere gegen ein Verrutschen und/oder Verdrehen, gesichert werden. Dies kann beispielsweise dadurch erfolgen, dass eine Kraft und/oder Spannung auf die Hilfsmittel ausgeübt wird, was beispielsweise durch eine entsprechende Formung des ersten Bauteils und/oder des zweiten Bauteils gewährleistet werden kann. Diese Kraft und/oder Spannung und/oder Verformung kann beispielsweise ein Verbiegen flexibler Hilfsmittel, beispielsweise flexibler Lanzetten und/oder Microsampler, insbesondere Metall-Lanzetten in Form von Flach-Lanzetten, bewirken. Die Spannung und/oder Kraft kann beispielsweise bei einer Probennahmebewegung aufgehoben werden und/oder durch den Aktor überwunden werden, beispielsweise indem der Aktor eine höhere Kraft und/oder höhere Spannung bereitstellt.

Das erfindungsgemäße Verfahren kann weiterhin einen Verfahrensschritt umfassen, in welchem mindestens eine Testchemie aufgebracht wird. Diese Testchemie kann beispielsweise auf das erste Bauteil und/oder das zweite Bauteil und/oder ein drittes, noch nicht genanntes Bauteil, insbesondere einen Träger, erfolgen. Das Aufbringen der Testchemie kann beispielsweise nach dem Aufbringen des zweiten Bauteils erfolgen, kann jedoch, alternativ oder zusätzlich, auch in einem vorgelagerten Verfahrensschritt und/oder gleichzeitig erfolgen. Die Testchemie ist, wie oben dargestellt, eingerichtet, um bei Anwesenheit mindestens eines nachzuweisenden Analyten mindestens eine messbare Eigenschaft zu ändern, beispielsweise eine optisch und/oder elektrisch messbare Eigenschaft. Diesbezüglich kann insbesondere auf die obige Beschreibung verwiesen werden.

Das Aufbringen der Testchemie erfolgt dabei derart, dass jeweils mindestens ein Bereich der Testchemie den Innenräumen der Kammern zuweist. Beispielsweise können jeweils ein oder mehrere Bereiche der Testchemie jeweils genau einer Kammer zugeordnet werden. Dies kann beispielsweise, wie oben dargestellt, dadurch erfolgen, dass jede Kammer, beispielsweise in ihrer Kammerwand, mindestens eine Testelementöffnung aufweist, auf welche von außen die Testchemie aufgebracht wird und/oder in welche die Testchemie zumindest teilweise eingebracht wird, so dass jeweils mindestens ein Bereich dem Innenraum der jeweiligen Kammer zuweist. Auf diese Weise können aus den Bereichen der Testchemie, die jeweils einer Kammer zuweisen, jeweils ein oder mehrere Testfelder entstehen, welche Bestandteil des analytischen Hilfsmittels sein können, und welche insbesondere ein- oder mehrere Teil-Hilfsmittel der analytischen Hilfsmittel bilden können. Die Bereiche der Testchemie, welche den Innenräumen der Kammern zuweisen, sollen dabei vorzugsweise vom Inneren der Kammern aus zugänglich sein.

Das Aufbringen der mindestens einen Testchemie kann beispielsweise mittels mindestens eines Trägers erfolgen. So kann beispielsweise ein Träger in Form einer oder mehrerer Scheiben und/oder Folien und/oder sonstiger Bauteile und/oder Bauelemente vorgesehen sein, auf welchen die Testchemie aufgebracht ist und welcher derart aufgebracht wird, dass die Testchemie den Kammern zuweist. Der Träger kann anschließend entfernt werden und/oder kann auch ganz oder teilweise Bestandteil des analytischen Magazins bleiben.

Nach dem Aufbringen der Testchemie kann diese optional zusätzlich bedeckt werden, wobei diese Testchemie beispielsweise feuchtigkeitsdicht im analytischen Magazin verschlossen und/oder aufgenommen wird. Auf diese Weise können beispielsweise auch nichtfeuchtigkeitsstabile Testchemien verwendet werden. Insbesondere wenn feuchtigkeitsstabile Testchemien verwendet werden, kann jedoch auf eine derartige Bedeckung der Testchemie auch vollständig verzichtet werden.

Es lassen sich analytische Magazine realisieren, bei welchen die Kammern analytische Hilfsmittel mit mindestens einer Lanzette und mindestens einem Testelement in Form beispielsweise mindestens eines Testfeldes umfassen. So kann beispielsweise mittels der mindestens einen Lanzette und/oder eines Sammelelements bzw. Transferelements bei einer Probennahmebewegung eine Probe der Körperflüssigkeit erzeugt und/oder aufgenommen werden und ins Innere der Kammer transferiert werden. Dieser Transfer kann, wie oben dargestellt, beispielsweise durch eine Rückbewegung als Bestandteil der Probennahmebewegung erfolgen, wobei ein beispielsweise auf der Lanzette und/oder dem Sammelelement und/oder dem Transferelement aufgenommener Teil der Probe ins Innere der Kammer transferiert wird. Alternativ oder zusätzlich kann die Aufnahme und/oder der Transfer, wie oben dargestellt, auch beispielsweise durch eine Kapillarwirkung mindestens eines Kapillarelements des analytischen Hilfsmittels erfolgen, welcher einen Transfer ins Innere der jeweils gerade verwendeten Kammer vornimmt. Der Transfer kann insbesondere derart ausgestaltet sein, dass bei diesem die aufgenommene Probe vollständig oder teilweise auf das mindestens eine Testelement übertragen wird, insbesondere auf ein oder mehrere Testfelder, beispielsweise das oben beschriebene mindestens eine durch Aufbringen der Testchemie erzeugte Testfeld. Hierzu kann das analytische System, welches das analytische Magazin verwendet, auch zusätzlich einen oder mehrere Aktoren umfassen, welche eingerichtet sind, um den Transfer der Probe von dem analytischen Hilfsmittel, beispielsweise einer Lanzette und/oder einem Microsampler, auf das mindestens eine Testelement, beispielsweise ein Testfeld, zu unterstützen. Beispielsweise kann ein Aktor vorgesehen sein, welche in die Kammer eingreift und eine mit der Probe beladene Lanzette und/oder einen Microsampler auf ein Testfeld drückt.

Die oben beschriebene Verfahrensvariante bzw. Variante des Testelementmagazins, bei welcher die Testchemie derart ausgestaltet ist, dass mindestens ein Bereich der Testchemie den Innenräumen der Kammer zuweist, insbesondere in Form eines oder mehrerer Testfelder pro Kammer, kann insbesondere derart durchgeführt werden, dass die Testchemie für mehrere, vorzugsweise für alle Kammern, gemeinsam aufgebracht wird. So kann beispielsweise die mindestens eine Testchemie in Form eines oder mehrerer Testchemiefelder aufgebracht werden, insbesondere in Form eines oder mehrerer durchgehende Testchemiefelder. Unter einem Testchemiefeld ist dabei eine durchgängig oder nicht-durchgängig mit Testchemie beschichtete Fläche, welche auch mehrere nicht-zusammenhängende Teil-Flächen umfassen kann, zu verstehen. Dieses gemeinsame Testchemiefeld, welches für mehrere oder vorzugsweise alle Kammern gemeinsam vorgesehen ist, kann beispielsweise in Form eines rechteckigen, runden oder grundsätzlich beliebig gestalteten Feldes ausgeführt sein. Dieses Testchemiefeld kann beispielsweise auf dem oben beschriebenen Träger aufgebracht sein, beispielsweise einer Trägerfolie und/oder einem anderen Bauelement. Der Träger kann beispielsweise ein Kunststoffmaterial, beispielsweise eine Kunststofffolie, und/oder ein Papiermaterial und/oder ein keramisches Material und/oder ein metallisches Material umfassen oder eine Kombination der genannten und/oder anderer Materialien. Insbesondere kann dabei ein durchgehender und vorzugsweise einstückiger Träger verwendet werden.

Insbesondere kann das Testchemiefeld, was besonders für runde analytische Magazine bevorzugt ist, rund oder kreisringförmig ausgestaltet sein. So kann beispielsweise mindestens ein Chemiering vorgesehen seien, welcher einen kreisringförmigen Träger, vorzugsweise einen durchgehenden und insbesondere einstückigen Träger (beispielsweise einen Trägerring), sowie mindestens ein darauf aufgebrachtes Testchemiefeld, vorzugsweise ein durchgehendes Testchemiefeld, aufweist. Alternativ können jedoch beispielsweise auch eine auf andere Weise gestaltete Testchemiescheibe und/oder ein Testchemieband vorgesehen sein, mit einem entsprechend gestalteten Träger, vorzugsweise einem durchgehenden und/oder einstückigen Träger, und mindestens einem darauf aufgebrachten Testchemiefeld. Auch andere Ausgestaltungen sind jedoch möglich, welche an die jeweilige Form des analytischen Magazins angepasst sein können. Das Testchemiefeld soll die Bereiche der Testchemie für mehrere Kammern, vorzugsweise für alle Kammern gleichzeitig, bereitstellen, insbesondere die Testfelder für die jeweiligen Kammern. Sind mehrere Testfelder mit unterschiedlichen Testchemien pro Kammer vorgesehen, so kann beispielsweise für jede Art von Testchemie ein separates Testchemiefeld für mehrere oder vorzugsweise alle Kammern bereitgestellt bzw. aufgebracht werden. Diese unterschiedlichen Arten von Testchemiefeldern können auf getrennten Trägern oder auch auf gemeinsamen Trägern vorgesehen sein. Vorzugsweise ist auch im Fall mehrerer Testchemiefelder und/oder mehrerer Arten von Testchemiefeldern ein gemeinsamer Träger, insbesondere ein einstückiger Träger, vorgesehen. Insbesondere kann der Träger auch durchgängig mit der Testchemie bedeckt sein, also derart, dass das Testchemiefeld nicht für die einzelnen Kammern unterbrochen wird, sondern einstückig für die mehreren Kammern, vorzugsweise für alle Kammern, ausgebildet ist. Alternativ kann aber auch ein nicht-durchgängig mit Testchemie beschichteter Träger verwendet werden. Der Träger selbst ist vorzugsweise selbst jedoch einstückig ausgestaltet, beispielsweise als einstückiger Trägerring.

Das Verfahren kann, wie oben dargestellt, weitere Verfahrensschritte umfassen, insbesondere Verfahrensschritte, bei welchen die Kammern vollständig oder teilweise, einzeln, gruppenweise oder insgesamt, versiegelt werden. Zu diesem Zweck kann in mindestens einem weiteren Verfahrensschritt mindestens eine Versiegelung auf mindestens eine Öffnung der Kammern aufgebracht werden. Sind pro Kammer mehrere Öffnungen vorgesehen, beispielsweise die oben genannten Öffnungen, so können diese einzeln oder in Gruppen oder gemeinsam verschlossen, bzw. versiegelt werden. Dabei kann die Versiegelung beispielsweise auch derart aufgebracht werden, dass für jede Art von Öffnungen für mehrere oder vorzugsweise alle Kammern gleichzeitig eine Versiegelung erfolgt. Unter einer Versiegelung ist dabei allgemein ein Verschließen der Öffnungen zu verstehen, welches, zumindest im Rahmen üblicher Benutzungsdauern bzw. Lagerdauern für das analytische Magazin, ein Eindringen von Umwelteinflüssen, insbesondere Luftfeuchtigkeit und/oder Keimen, ins Innere der Kammern zumindest weitgehend verhindert. Auf diese Weise kann beispielsweise eine gleich bleibende Qualität der analytischen Hilfsmittel über eine vorgegebene Lagerdauer, beispielsweise eine Lagerdauer von einigen Monaten bis hin zu einigen Jahren, gewährleistet sein.

Die Versiegelung kann beispielsweise durch mindestens ein Versiegelungselement erfolgen, welches vorzugsweise derart ausgestaltet ist, dass dieses die jeweilige Funktion bzw. den Verwendungszweck der jeweiligen mindestens einen Öffnung nicht beeinträchtigt. Beispielsweise kann die Versiegelung bei der mindestens einen Probennahmeöffnung derart ausgestaltet sein, dass diese für die Probennahmebewegung durch das analytische Hilfsmittel und/oder ein weiteres Element des analytischen Magazins und/oder des analytischen Systems geöffnet werden kann, beispielsweise durch Durchstechen und/oder durch Schneiden. Entsprechend kann beispielsweise die mindestens eine Aktoröffnung ausgestaltet sein, dass diese für eine Aktorbewegung im Rahmen der Probennahmebewegung geöffnet wird, beispielsweise durch den Aktor selbst und/oder ein weiteres Element des analytischen Magazins und/oder des analytischen Systems. Ist mindestens eine Messöffnung vorgesehen, so kann die Versiegelung dieser Messöffnung beispielsweise derart ausgestaltet sein, dass die Messöffnung für eine Messung freigelegt wird. Alternativ oder zusätzlich kann, je nach Art der Messung, die Versiegelung auch beispielsweise derart ausgestaltet sein, dass diese beispielsweise eine optische Messung ermöglicht, zu welchem Zweck die Versiegelung der mindestens einen Messöffnung beispielsweise transparent für Detektionslicht und/oder Anregungslicht ausgestaltet sein kann.

Eine Sonderrolle kann die mindestens eine optionale Testelement-Öffnung einnehmen. Diese Testelementöffnung kann beispielsweise bereits beim Aufbringen bzw. durch das Aufbringen der Testchemie gemäß der obigen Beschreibung verschlossen und/oder versiegelt werden. Zusätzlich kann mindestens eine Versiegelung auf diese mindestens eine Testelementöffnung aufgebracht sein, beispielsweise um verbleibende Zwischenräume zu versiegeln.

Die Versiegelung kann ein oder mehrere Versiegelungselemente umfassen, welche den oben beschriebenen Zwecken angepasst sein können und welche auch für mehrere Öffnungen einstückig ausgebildet sein können. Beispielsweise können entsprechende Versiegelungsfolien vorgesehen sein, beispielsweise dünne Kunststofffolien und/oder Metallfolien. Derartige Versiegelungselemente sind aus dem Stand der Technik grundsätzlich bekannt.

Wie oben dargestellt, wird neben dem vorgeschlagenen Verfahren in einer oder mehreren der beschriebenen Verfahrensvarianten weiterhin ein analytisches Magazin vorgeschlagen. Dieses analytische Magazin kann beispielsweise nach einem Verfahren gemäß einer oder mehreren der beschriebenen Verfahrensvarianten herstellbar sein, wobei jedoch grundsätzlich auch andere Herstellungsverfahren einsetzbar sind. Dieses analytische Magazin umfasst eine Mehrzahl von in Kammern aufgenommenen analytischen Hilfsmitteln. Das analytische Magazin umfasst weiterhin mindestens eine Testchemie, welche eingerichtet ist, um bei Anwesenheit mindestens eines nachzuweisenden Analyten mindestens eine messbare Eigenschaft zu ändern.

Weiterhin wird vorgeschlagen, den oben beschriebenen Aspekt der gemeinsamen Aufbringung der Testchemie für mehrere Kammern, vorzugsweise für alle Kammern, auch ggf. unabhängig von dem genannten Herstellungsverfahren zu realisieren. Dementsprechend kann die mindestens eine Testchemie auf einen durchgehenden Träger aufgebracht sein und mindestens in Testchemiefeld im Sinne der obigen Definition bilden. Insbesondere können das mindestens eine Testchemiefeld und der durchgehende Träger mindestens einen Chemiering und/oder mindestens ein Testchemieband bilden. Diesbezüglich kann auf die obige Beschreibung verwiesen werden.

Das Testchemiefeld ist dabei auf einen durchgehenden Träger aufgebracht. Unter einem durchgehenden Träger ist dabei ein Träger zu verstehen, welcher Testchemie für mehrere und vorzugsweise für alle Kammern gleichzeitig trägt. Insbesondere kann der Träger einstückig ausgestaltet sein, beispielsweise als Trägerring. Bezüglich der möglichen Ausgestaltungen des Trägers kann beispielsweise auf die obige Beschreibung des Verfahrens verwiesen werden.

Dieses mindestens eine Testchemiefeld, welches, wie oben dargestellt, ein oder auch mehrere Teil-Felder, auch nicht-zusammenhängende Teil-Felder, aufweisen kann, stellt dabei jeweils mindestens einen Bereich des Testchemiefeldes an die Kammern bereit, wobei dieser jeweils mindestens eine Bereich des Testchemiefeldes den Innenräumen der Kammern zuweist. Wie oben dargestellt, kann dieser mindestens eine Bereich somit insbesondere pro Kammer jeweils mindestens ein Testfeld erzeugen, welches einen Bestandteil der in den Kammern aufgenommenen analytischen Hilfsmittel bildet und/oder der Teil-Hilfsmittel dieser analytischen Hilfsmittel.

Das Testchemiefeld kann insbesondere Bestandteil eines Gehäuses des analytischen Magazins sein, insbesondere Bestandteil einer äußeren Magazingehäusewand. Beispielsweise kann dies, wie oben ausgeführt, dadurch erfolgen, dass das Testchemiefeld von außen auf eine Öffnung eines Gehäuseteils des Gehäuses aufgebracht wird, so dass das Testchemiefeld zumindest teilweise vom Inneren der Kammer aus zugänglich ist. Das Gehäuse kann beispielsweise, wie oben dargestellt, im Wesentlichen starr ausgebildet sein, also derart, dass dieses seine Form zumindest unter Einwirkung der eigenen Gewichtskräfte im Wesentlichen nicht ändert. Das Gehäuse kann beispielsweise die oben genannten Bauteile, also das mindestens eine erste Bauteil, das optional mindestens zweite Bauteil und ggf. ein oder mehrere weitere Bauteile, umfassen. Das Gehäuse kann beispielsweise ein oder mehrere Kunststoffwerkstoffe und/oder ein oder mehrere keramische Werkstoffe und/oder ein oder mehrere weitere Werkstoffe umfassen, beispielsweise thermoplastische Kunststoffwerkstoffe, duroplastische Kunststoffwerkstoffe, ggf. mit entsprechenden Füllstoffen oder Kombinationen der genannten und/oder anderer Materialien.

Das beschriebene Verfahren und/oder das beschriebene analytische Magazin in einer oder mehreren der beschriebenen Ausführungsformen weist, wie oben bereits angedeutet, zahlreiche Vorteile gegenüber bekannten Verfahren und Vorrichtungen auf. Insbesondere kann das analytische Magazin für so genannte Micro-Sampler verwendet werden, also analytische Systeme, bei welchen die Probenerzeugung und die Probennahme sowie optional auch die Probenanalyse innerhalb eines einzelnen integrierten Systems erfolgt. Vorzugsweise können dabei kleine Probenvolumina aufgenommen werden, beispielsweise Probenvolumina unterhalb von 1 µl, insbesondere unterhalb von 500 µl.

Der Herstellungsprozess kann im Wesentlichen auf die Handhabung von wenigen Bauelementen beschränkt werden. In einem ersten Verfahrensschritt können beispielsweise, gemäß einer der oben beschriebenen Verfahrensvarianten, Strukturen einzelner Lanzetten, beispielsweise Nadelelemente, geätzt werden, beispielsweise aus einem Metallblech. Auf diese Weise kann beispielsweise eine Metallscheibe erzeugt werden (Metal Disc), welche das Halteelement und die Lanzetten umfasst. Mittels der Metallscheibe können die einzelnen Lanzetten untereinander verbunden sein. Das Metallblech kann anschließend beispielsweise in das erste Bauteil, beispielsweise ein Kunststoffbauteil eines vollständig oder teilweise aus Kunststoff hergestellten Gehäuses, eingelegt und/oder auf dieses aufgelegt werden. Beim anschließenden Vereinzeln der Lanzetten werden diese vorzugsweise direkt geordnet alle auf einmal in die Kammern des Gehäuses abgelegt, vorzugsweise ohne dass hierfür eine gesonderte Ausrichtung der Lanzetten erforderlich ist. Auf diese Weise kann beispielsweise auf eine Handhabung einzelner, insbesondere miniaturisierter, Disposables beim Einführen in die jeweiligen Magazinkammem verzichtet werden. In einem nächsten Schritt kann das Gehäuse durch ein weiteres Gehäuseteil vervollständigt werden, wobei die nun vereinzelten Lanzetten beispielsweise in ihren Kammern gehalten werden. Anschließend kann, wie oben dargestellt, das Magazingehäuse beispielsweise mit einem Chemiering abgedeckt werden, welcher bevorzugterweise ein durchgängiges Testchemiefeld aufweist. Insgesamt lassen sich auf diese Weise die Herstellungskosten und der Aufwand der Herstellung erheblich reduzieren.

Weiterhin lässt sich mittels des vorgeschlagenen Verfahrens auch eine Querkontamination der analytischen Hilfsmittel innerhalb der Kammern zuverlässig verhindern. Beispielsweise kann dies durch ein oder mehrere der oben beschriebenen Verbindungsverfahren gewährleistet werden, mittels derer mehrere Gehäuseteile, beispielsweise das erste Bauteil und das zweite Bauteil, miteinander verbunden werden, wobei insbesondere die einzelnen Kammern voneinander getrennt werden können. Dies kann insbesondere durch ein Laserschweißverfahren erfolgen, wobei vorzugsweise benachbarte Kammern durch durchgehende Schweißnähte voneinander getrennt werden.

Insbesondere die einstückige Herstellung der analytischen Hilfsmittel mit dem Halteelement bzw. von Teil-Hilfsmitteln mit dem Halteelement weist zahlreiche Vorteile auf. So können beispielsweise spezielle Ätzstrukturen für die Lanzetten beziehungsweise Microsampler verwendet werden, über welche die Lanzetten mit dem Halteelement, beispielsweise einem Metallblech und/oder Metallrahmen, verbunden werden können. Diese Ätzstrukturen können beispielsweise, wie oben dargestellt, Sollbruchstellen und/oder Verjüngungen aufweisen, so dass beispielsweise beim Herausbrechen und/oder anderweitigen Trennen der einzelnen Lanzetten aus dem Halteelement keine Abbruchreste bestehen bleiben, welche ein Gleiten der Lanzetten innerhalb der Kammern behindern könnten. Auf diese Weise kann eine hohe Prozesssicherheit gewährleistet werden.

Durch die bevorzugte starre Ausgestaltung des Testelementmagazins und/oder die bevorzugte gemeinsame Einbringung der analytischen Hilfsmittel in mehrere, vorzugsweise alle, Kammern durch ein entsprechendes Halteelement lassen sich auch Vorteile beispielsweise gegenüber durch ein Band oder ähnliche flexible Elemente verbundenen analytischen Hilfsmittel und der Herstellung entsprechender analytischer Magazine erzielen. Eine Handhabung von Bändern ist in diesem Fall nicht erforderlich. Gleichwohl ist die starre Ausgestaltung jedoch nicht zwingend notwendig, da beispielsweise das Magazin und/oder das erste Bauteil und/oder das Halteelement auch ganz oder teilweise flexibel ausgestaltet sein können, beispielsweise in Form von Folien, Bändern, Ketten, oder ähnlichem.

Das analytische Magazin umfasst eine Mehrzahl von analytischen Hilfsmitteln. Das analytische Magazin weist mindestens zwei Kammern auf, in denen die analytischen Hilfsmittel aufnehmbar sind. Die analytischen Hilfsmittel sind dabei in mindestens einer der Kammern aufgenommen. Dabei sind grundsätzlich zwei Prinzipien der Aufnahme in den Kammern denkbar. So kann beispielsweise pro Kammer jeweils ein analytisches Hilfsmittel aufgenommen sein, insbesondere ein analytisches Hilfsmittel, welches mindestens ein Teil-Hilfsmittel in Form eines Testelements mit einer Testchemie umfasst. Optional kann, vorzugsweise zusätzlich, pro Kammer mindestens ein weiteres Teil-Hilfsmittel in Form einer Lanzette und/oder eines Microsamplers vorgesehen sein. Bei diesem Prinzip können die analytischen Hilfsmittel insbesondere nach einer Verwendung in derselben Kammer remagaziniert werden. Alternativ ist jedoch auch eine Remagazinierung in einer anderen Kammer möglich. Dieses erste Prinzip ist insbesondere bei scheibenförmigen oder stangenförmigen Magazinen bevorzugt. Nach einem weiteren Prinzip können mindestens eine erste Kammer für unbenutzte analytische Hilfsmittel und mindestens eine zweite Kammer für benutzte analytische Hilfsmittel vorgesehen sein. In diesem Fall können beispielsweise für eine Benutzung die analytischen Hilfsmittel aus der ersten Kammer entnommen und nach der Benutzung in die zweite Kammer überführt werden, welche räumlich getrennt von der ersten Kammer ausgebildet sein kann. Dieses Prinzip kann beispielsweise für Bandmagazine eingesetzt werden, wobei beispielsweise in einer ersten Kammer ein Gutwickel zur Aufnahme unbenutzter analytischer Hilfsmittel vorgesehen ist und in einer zweiten Kammer ein Schlechtwickel zur Aufnahme benutzter analytischer Hilfsmittel.

Die analytischen Hilfsmittel umfassen jeweils mindestens ein Testelement mit mindestens einer Testchemie zum Nachweis mindestens eines Analyten in einer flüssigen Probe, insbesondere einer Körperflüssigkeit. Die analytischen Hilfsmittel werden dabei häufig auch als "Tests" bezeichnet, unabhängig von deren Funktion und Ausgestaltung. Unter einem Test kann dabei also allgemein mindestens analytisches Hilfsmittel verstanden werden, welches für einen Testvorgang genutzt werden kann. Dabei kann es sich beispielsweise um ein Testelement oder um eine Lanzette oder auch um ein Paar aus einem Testelement und einer Lanzette handeln, wobei vorzugsweise genau ein Test in genau einer Kammer gelagert ist. Ein Test kann also beispielsweise mehrere zusammengehörige Teil-Hilfsmittel umfassen. Ein Test kann beispielsweise in genau einer Kammer aufgenommen sein. Im Rahmen der vorliegenden Erfindung wird hier und im Folgenden jedoch zwischen einem Test und einem analytischen Hilfsmittel sprachlich und inhaltlich nicht weiter unterschieden, einschließlich der Möglichkeit, dass ein Test mehrere Teil-Hilfsmittel umfassen kann, beispielsweise jeweils ein Testelement und eine Lanzette.

Bei herkömmlichen analytischen Magazinen und Testelementen, insbesondere für den Nachweis von Glukose, wird in der Regel eine Testchemie verwendet, welche empfindlich ist gegenüber Luftfeuchtigkeit und welche bei zu langer Exposition an Luft ihre Funktion verschlechtern oder sogar ganz einbüßen kann. Dementsprechend müssen beispielsweise herkömmliche Teststreifen in gegenüber Luftfeuchtigkeit dichten Behältern gelagert werden. Diese Behälter sind üblicherweise mit einem Trockenmittel, also einem Feuchtigkeit absorbierenden Material, beispielsweise Aktivkohle, teilbefüllt. Werden nun bei integrierten Systemen analytische Magazine und/oder analytische Hilfsmittel, beispielsweise Einweg-Hilfsmittel (Disposables), entwickelt, in denen Testelemente einzeln oder in Gruppen verpackt werden, so müssen auch diese Verpackungen feuchtigkeitsdicht ausgestaltet sein. Diese Forderung nach Dichtigkeit gegenüber Feuchtigkeit schränkt jedoch die Auswahl potentieller Materialien extrem ein, insbesondere potentieller Materialien für das Gehäuse. Dies ist insbesondere dadurch bedingt, dass in der Regel zusätzliche Anforderungen existieren, die gleichzeitig erfüllt sein müssen. So müssen die verwendeten Materialien in den meisten Fällen sterilisierbar sein, insbesondere mittels ionisierender Strahlung. Alternativ oder zusätzlich dürfen die verwendeten Materialien in der Regel nicht ausgasen, insbesondere nicht nach oder bei einer Strahlenbelastung durch einen Sterilisationsprozess. Wiederum alternativ oder zusätzlich müssen die verwendeten Materialien für den gewählten Herstellungsprozess geeignet sein, beispielsweise für ein Spritzgießverfahren und/oder einen anderen Formgebungsprozess. Wiederum alternativ oder zusätzlich sollten die verwendeten Materialien vorzugsweise biokompatibel sein und/oder sollten fügbar und/oder versiegelungsfähig sein. Weitere Anforderungen können existieren. Dabei ist insbesondere die Anforderung einer Dichtigkeit gegenüber Feuchtigkeit eine in der Praxis schwer zu erfüllende Forderung, da die meisten Kunststoffe diffusionsoffen für Feuchtigkeit sind, insbesondere bei geringen Wandstärken, beispielsweise Wandstärken von weniger als einem Millimeter. Die Testchemie wird dabei derart auszugestalten, dass diese zumindest weitgehend stabil gegenüber Umwelteinflüssen, insbesondere gegenüber Feuchtigkeit, ist. Die Testchemie kann insbesondere als Trockenchemie vorliegen, insbesondere auf einem Teststreifen. Im Rahmen der vorliegenden Erfindung wird unter einer im Wesentlichen gegenüber Umwelteinflüssen stabilen Testchemie eine Testchemie verstanden, die gegenüber Luftfeuchtigkeit und vorteilhafterweise ebenfalls gegenüber Sterilisationsverfahren, insbesondere Sterilisationsverfahren unter Verwendung ionisierender Strahlung, stabil ist. Hierbei wird als stabil bezeichnet, wenn bei einer Lagerung bei 32 °C, einer relativen Luftfeuchtigkeit von 85% bei Normaldruck über eine Zeitdauer von drei Wochen die Aktivität, beispielsweise die Enzymaktivität der Testchemie des analytischen Hilfsmittels, um weniger als 50%, vorzugsweise um weniger als 30% und besonders bevorzugt um weniger als 20% abnimmt. Die Aktivität kann dabei grundsätzlich mittels eines beliebigen, aus dem Stand der Technik bekannten Verfahrens bestimmt werden, da im Rahmen der angegebenen Definition lediglich ein Verhältnis der Abnahme der mit diesem Verfahren gemessenen Aktivität zu einer mit diesem Verfahren gemessenen Aktivität vor der Lagerung bzw. unmittelbar nach der Herstellung des analytischen Hilfsmittels von Relevanz ist. Die Aktivität kann sich dabei insbesondere auf eine Enzymaktivität einer Trockenchemie beziehen, insbesondere in einem Teststreifen. Beispielsweise sind Verfahren bekannt, die zur Messung der Enzymaktivität das Enzym aus der Testchemie bzw. dem Teststreifen extrahieren und anschließend beispielsweise mittels einer Ultraviolett-Absorption die Aktivität bestimmen. Diesbezüglich kann beispielsweise auf H. U. Bergmeyer: Methoden der enzymatischen Analyse, Verlag Chemie, 2. Auflage 1970, S. 417 oder auf Banauch et al.: A glucose dehydrogenase for the determination of glucose concentrations in body fluids, Z. Klin. Chem. Klin. Biochem. 1975 Mar; 13(3):101-7 verwiesen werden. Beispielsweise kann für den Test ein Teststreifen mit der Testchemie hergestellt werden, mit einem üblichen Verfahren die Enzymaktivität eines Enzyms der Testchemie gemessen werden, dann die oben beschriebene Lagerung durchgeführt werden und anschließend erneut dasselbe Verfahren zur Messung der Enzymaktivität durchgeführt werden. Diese Prozedur wird üblicherweise mit einem repräsentativen Kollektiv an Teststreifen bzw. Testchemien durchgeführt. Alternativ oder zusätzlich zu einer Stabilität gegenüber Umwelteinflüssen in Form von Luftfeuchtigkeit kann vorzugsweise auch eine hohe Stabilität der Testchemie gegenüber Umwelteinflüssen in Form von üblicherweise für eine Sterilisierung der analytischen Hilfsmittel und/oder der analytischen Magazine insgesamt verwendeten Strahlung gegeben sein, beispielsweise einer Gammastrahlung und/oder Betastrahlung und/oder einer anderen Art von ionisierender Strahlung.

Als Beispiel einer derartigen, gegenüber Umwelteinflüssen stabilen Testchemie kann auf die oben bereits zitierte WO 2007/012494 A1 verwiesen werden. Die dort dargestellte Testchemie ist auch im Rahmen der vorliegenden Erfindung einsetzbar, allein oder auch in Kombination mit einer oder mehreren anderen Testchemien. Alternativ oder zusätzlich kann die Testchemie auch ausgestaltet sein wie in der nachveröffentlichten europäischen Patentanmeldung mit der Nummer EP 08003054.7 oder in der nachveröffentlichten internationalen Patentanmeldung mit der Nummer PCT/EP2009/001206 aus dem Hause der Anmelderin der vorliegenden Patentanmeldung beschrieben.

So kann die Testchemie beispielsweise ein Enzym und ein stabiles Coenzym enthalten, welche gemeinsamen gelagert sind. Überraschenderweise wurde gefunden, dass mit Hilfe eines stabilen Coenzyms eine Langzeitstabiliserung von mehreren Wochen bzw. Monaten bei hoher relativer Feuchte oder sogar in flüssiger Phase und bei erhöhten Temperaturen möglich ist. Diese Erkenntnis ist überraschend, da bekannt ist, dass Enzyme in Gegenwart von nativem Coenzym zwar eine erhöhte Kurzzeitstabilität für einige Stunden besitzen, aber eine geringere Haltbarkeit über einen längeren Zeitraum aufweisen. Gegenüber diesen Erkenntnissen gegenüber dem Stand der Technik war es überraschend, dass ein Enzym in Gegenwart eines stabilen Coenzyms eine deutlich erhöhte Langzeitstabilität als ein Enzym in Gegenwart eines nativen Coenzyms besitzt, insbesondere da die stabilen Coenzyme eine niedrigere Bindungskonstante mit dem Enzym als das native Coenzym besitzen.

Das stabilisierte Enzym kann insbesondere ein Coenzym-abhängiges Enzym sein. Geeignete Enzyme sind z.B. Dehydrogenasen, ausgewählt aus einer Glucose-Dehydrogenase (E.C.1.1.1.47), Lactat-Dehydrogenase (E.C.1.1.1.27, 1.1.1.28), Malat-Dehydrogenase (E.C.1.1.1.37), Glycerin-Dehydrogenase (E.C.1.1.1.6), Alkohol-Dehydrogenase (E.C.1.1.1.1), alpha-Hydroxybutyrat-Dehydrogenase, Sorbitol-Dehydrogenase oder Aminosäure-Dehydrogenase, z. B. L-Aminosäure-Dehydrogenase (E.C.1.4.1.5). Weitere geeignete Enzyme sind Oxidasen, wie etwa Glucoseoxidase (E.C.1.1.3.4) oder Cholesterinoxidase (E.C.1.1.3.6) bzw. Aminotransferasen, wie z.B. Aspartat oder Alanin Aminotransferase, 5'-Nukleotidase oder Creatin-Kinase. Vorzugsweise ist das Enzym Glucose-Dehydrogenase.
Als besonders bevorzugt hat sich der Einsatz einer mutierten Glucose-Dehydrogenase erwiesen. Der Begriff "Mutante", wie er im Rahmen der vorliegenden Anmeldung verwendet wird, bezeichnet eine genetisch veränderte Variante eines nativen Enzyms, welche bei gleicher Anzahl an Aminosäuren eine gegenüber dem Wildtyp-Enzym veränderte Aminosäuresequenz besitzt, d.h. sich in mindestens einer Aminosäure vom Wildtyp-Enzym unterscheidet. Die Einführung der Mutation(en) kann ortsspezifisch oder nicht-ortsspezifisch, bevorzugt ortsspezifisch unter Verwendung von im Fachbereich bekannten rekombinanten Methoden, erfolgen, wobei entsprechend den jeweiligen Anforderungen und Bedingungen mindestens ein Aminosäureaustausch innerhalb der Aminosäuresequenz des nativen Enzyms resultiert. Besonders bevorzugt weist die Mutante eine gegenüber dem Wildtyp-Enzym erhöhte thermische oder hydrolytische Stabilität auf.
Die mutierte Glucose-Dehydrogenase kann die gegenüber der korrespondierenden Wildtyp-Glucose-Dehydrogenase veränderte(n) Aminosäure(n) grundsätzlich an einer beliebigen Position ihrer Aminosäuresequenz enthalten. Vorzugsweise umfasst die mutierte Glucose-Dehydrogenase eine Mutation an mindestens einer der Positionen 96, 170 und 252 der Aminosäuresequenz der Wildtyp-Glucose-Dehydrogenase, wobei Mutanten mit Mutationen an Position 96 und Position 170 bzw. Mutationen an Position 170 und Position 252 besonders bevorzugt sind. Als vorteilhaft hat es sich erwiesen, wenn die mutierte Glucose-Dehydrogenase neben diesen Mutationen keine weiteren Mutationen enthält.

Die Mutation an den Positionen 96, 170 und 252 kann grundsätzlich einen beliebigen Aminosäureaustausch umfassen, welcher zu einer Stabilisierung, z.B. einer Erhöhung der thermischen oder hydrolytischen Stabilität, des Wildtyp-Enzyms führt. Vorzugsweise umfasst die Mutation an Position 96 einen Aminosäureaustausch von Glutaminsäure gegen Glycin, während in Bezug auf Position 170 ein Aminosäureaustausch von Glutaminsäure gegen Arginin oder Lysin, insbesondere ein Aminosäureaustausch von Glutaminsäure gegen Lysin, bevorzugt ist. Was die Mutation an Position 252 anbetrifft, so umfasst diese bevorzugt einen Aminosäureaustausch von Lysin gegen Leucin.

Die mutierte Glucose-Dehydrogenase kann durch Mutation einer aus einer beliebigen biologischen Quelle stammenden Wildtyp-Glucose-Dehydrogenase erhalten werden, wobei der Begriff "biologische Quelle" im Sinne dieser Erfindung sowohl Prokaryoten, wie beispielsweise Bakterien, als auch Eukaryoten, wie beispielsweise Säuger und andere Tiere, umfasst. Vorzugsweise entstammt die Wildtyp-Glucose-Dehydrogenase einem Bakterium, wobei es sich besonders bevorzugt um eine Glucose-Dehydrogenase aus Bacillus megaterium, Bacillus subtilis oder Bacillus thuringiensis, insbesondere aus Bacillus subtilis, handelt.

In einer besonders bevorzugten Ausführungsform der vorliegenden Erfindung handelt es sich bei der mutierten Glucose-Dehydrogenase um eine durch Mutation von Wildtyp-Glucose-Dehydrogenase aus Bacillus subtilis erhaltene Glucose-Dehydrogenase, welche die in SEQ ID NO: 1 (GlucDH_E96G_E170K) oder die in SEQ ID NO: 2 (GlucDH_E170K_K252L) dargestellte Aminosäuresequenz besitzt.

Das stabile Coenzym ist vorzugsweise ein gegenüber dem nativen Coenzym chemisch verändertes Coenzym, welches eine im Vergleich zum nativen Coenyzm höhere Stabilität, (z.B. hydrolytische Stabilität) aufweist. Vorzugsweise ist das stabile Coenyzm unter Testbedingungen gegenüber Hydrolyse stabil. Im Vergleich zum nativen Coenzym kann das stabile Coenzym eine verringerte Bindungskonstante für das Enzym aufweisen, beispielsweise eine um den Faktor von 2 oder mehr verringerte Bindungskonstante.

Bevorzugte Beispiele für stabile Coenzyme sind stabile Derivate von Nicotinamid-Adenin-Dinukleotid (NAD/NADH) oder Nicotinamid-Adenin-Dinukleotidphosphat (NADP/NADPH), oder verkürzte NAD-Derivate, z.B. ohne AMP-Teil bzw. mit nicht nukleosidischen Resten, z.B. hydrophoben Resten. Ebenfalls bevorzugt ist als stabiles Coenzym im Sinne der vorliegenden Erfindung ist die Verbindung der Formel (I).

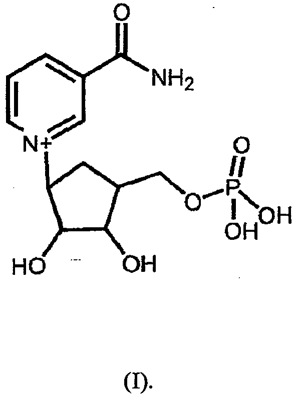

Bevorzugte stabile Derivate von NAD/NADH bzw. NADP/NADPH sind in den zuvor genannten Referenzen beschrieben, auf deren Offenbarung hiermit ausdrücklich Bezug genommen wird. Besonders bevorzugte stabilisierte Coenzyme sind in WO 2007/012494 bzw. US 11/460,366 beschrieben, auf deren Offenbarung hiermit ausdrücklich Bezug genommen wird. Das stabile Coenzym wird besonders bevorzugt aus Verbindungen mit der allgemeinen Formel (II) ausgewählt: 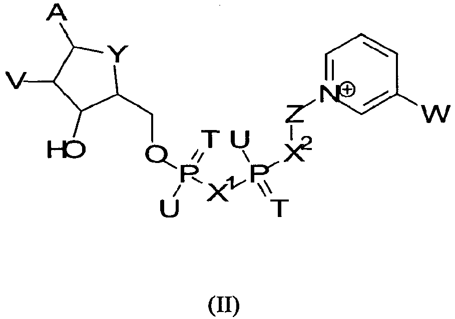 mit
A = Adenin oder ein Analog davon,
T = jeweils unabhängig O, S,
U = jeweils unabhängig OH, SH, BH₃⁻, BCNH₂⁻,
V = jeweils unabhängig OH oder eine Phosphatgruppe, oder zwei Gruppen, die eine cyclische Phosphatgruppe bilden;
W = COOR, CON(R)₂, COR, CSN(R)₂ mit R = jeweils unabhängig H oder C₁-C₂-Alkyl, X¹, X² = jeweils unabhängig O, CH₂, CHCH₃, C(CH₃)₂, NH, NCH₃,
Y = NH, S, O, CH₂,
Z = ein linearer oder cyclischer organischer Rest ist,
   mit der Maßgabe, dass Z und der Pyridin-Rest nicht durch eine glycosidische Verbindung verknüpft sind, oder ein Salz oder gegebenenfalls eine reduzierte Form davon.

In den Verbindungen der Formel (II) ist Z vorzugsweise ein linearer Rest mit 4-6 C-Atomen, vorzugsweise 4 C-Atomen, worin 1 oder 2 C-Atome gegebenenfalls durch ein oder mehrere Heteroatome ausgewählt aus O, S und N ersetzt sind, oder ein Rest umfassend eine cyclische Gruppe mit 5 oder 6 C-Atomen, die gegebenenfalls ein Heteroatom ausgewählt aus O, S und N sowie gegebenenfalls einen oder mehrere Substituenten enthält, und einen Rest CR⁴₂, wobei CR⁴₂ an die cyclische Gruppe und an X² gebunden ist, mit R⁴ = jeweils unabhängig H, F, Cl, CH₃.

Besonders bevorzugt ist Z ein gesättiger oder ungesättigter carbocyclischer oder heterocyclischer Fünfring, insbesondere eine Verbindung der allgemeinen Formel (III) 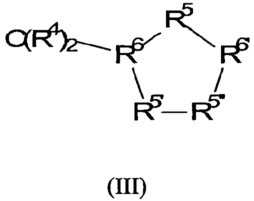
wobei zwischen R⁵' und R⁵" eine Einfach- oder Doppelbindung vorliegen kann, mit
R⁴ = jeweils unabhängig H, F, Cl, CH₃,
R⁵ =CR⁴₂,
wobei R⁵' = O, S, NH, NC₁-C₂-Alkyl, CR⁴₂, CHOH, CHOCH₃, und
R⁵" = CR⁴₂, CHOH, CHOCH₃, wenn zwischen R⁵' und R⁵" eine Einfachbindung vorliegt, und wobei R⁵'=R⁵"=CR⁴, wenn zwischen R⁵' und R⁵" eine Doppelbindung vorliegt, und
R⁶, R⁶' = jeweils unabhängig CH oder CCH₃.

In einer bevorzugten Ausführungsform enthalten die erfindungsgemäßen Verbindungen Adenin oder Adenin-Analoga, wie z.B. C₈- und N₆-substituiertes Adenin, Deaza-Varianten wie 7-Deaza, Azavarianten wie 8-Aza oder Kombinationen wie 7-Deaza oder 8-Aza oder carbocyclische Analoga, wie Formycin, wobei die 7-Deazavarianten in der 7-Position mit Halogen, C₁-C₆-Alkinyl, -Alkenyl oder -Alkyl substituiert sein können.

In einer weiteren bevorzugten Ausführungsform enthalten die Verbindungen Adenosin-Analoga, welche statt Ribose z.B. 2-Methoxy-deoxyribose, 2'-Fluoro-deoxyribose, Hexitol, Altritol bzw. polycyclische Analoga, wie Bicyclo-, LNA- und Tricyclo-Zucker enthalten.

Insbesondere können in den Verbindungen der Formel (II) auch (Di-)-Phosphatsauerstoffe isotronisch ersetzt sein, wie z.B. O⁻ durch S⁻ bzw. BH₃⁻, O durch NH, NCH₃ bzw. CH₂ und =O durch =S.

In den erfindungsgemäßen Verbindungen der Formel (II) ist W vorzugsweise CONH₂ oder COCH₃.

In den Gruppen der Formel (III) ist R⁵ vorzugsweise CH₂. Weiterhin ist bevorzugt, dass R⁵' ausgewählt ist aus CH₂, CHOH und NH. In einer besonders bevorzugten Ausführungsform sind R⁵' und R⁵" jeweils CHOH. In noch einer weiteren bevorzugten Ausführungsform ist R⁵' NH und R⁵" CH₂.

In der am stärksten bevorzugten Ausführungsform handelt es sich bei dem stabilen Coenzym um carbaNAD.

Die bevorzugte Testchemie ist insbesondere derart ausgestaltet, dass die in dieser enthaltene Enzyme Langzeit-stabilisiert sind. Dies bedeutet, dass man das mit einem stabilen Coenzym stabilisierte Enzym, z.B. als Trockensubstanz, beispielsweise über eine Dauer von mindestens zwei Wochen, vorzugsweise von mindestens vier Wochen und besonders bevorzugt von mindestens acht Wochen lagert und wobei die Enzymaktivität vorzugsweise um weniger als 50 %, besonders bevorzugt weniger als 30 % und am meisten bevorzugt um weniger als 20 % bezüglich des Ausgangswerts der Enzymaktivität abnimmt.

Weiterhin kann die Testchemie derart ausgestaltet werden, dass das mit einem stabilen Coenzym stabilisierte Enzym bei erhöhten Temperaturen, beispielsweise bei einer Temperatur von mindestens 20 °C, vorzugsweise von mindestens 25 °C und besonders bevorzugt von mindestens 30 °C gelagert wird. Die Enzymaktivität nimmt dabei vorzugsweise um weniger als 50 %, besonders bevorzugt weniger als 30 % und am meisten bevorzugt weniger als 20 % bezüglich ihres Ausgangswerts ab.

Durch die erfindungsgemäße Stabilisierung ist es möglich, das mit einem stabilen Coenzym stabilisierte Enzym auch ohne Trocknungsreagenz für eine lange Zeit, wie oben angegeben, und/oder bei hohen Temperaturen, wie oben angegeben, zu lagern. Weiterhin kann das stabilisierte Enzym auch bei einer hohen relativen Luftfeuchtigkeit, z.B. einer relativen Luftfeuchtigkeit von mindestens 50 % gelagert werden, wobei die Enzymaktivität vorzugsweise um weniger als 50 %, besonders bevorzugt weniger als 30 % und am meisten bevorzugt weniger als 20 % bezüglich des Ausgangswerts abnimmt.

Die Lagerung des mit einem stabilen Coenzym stabilisierten Enzyms kann einerseits als Trockensubstanz und andererseits in Flüssigphase erfolgen. Bevorzugt erfolgt die Lagerung des stabilisierten Enzyms auf oder in einem Testelement, das zur Bestimmung eines Analyten geeignet ist. Das mit einem stabilen Coenzym stabilisierte Enzym ist dabei Bestandteil der bevorzugten Testchemie, welche gegebenenfalls noch weitere Bestandteile wie etwa Salze, Puffer, etc. enthalten kann. Vorzugsweise ist die Testchemie dabei frei von einem Mediator.

Das mit einem stabilen Coenzym stabilisierte Enzym kann allgemein zum Nachweis von Analyten, beispielsweise Parametern in Körperflüssigkeiten wie etwa Blut, Serum, Plasma oder Urin bzw. in Abwasserproben oder Lebensmitteln eingesetzt werden.

Als Analyten können beliebige biologische oder chemische Substanzen bestimmt werden, die durch eine Redoxreaktion nachgewiesen werden können, z.B. Substanzen, bei denen es sich um Substrate eines Coenzym-abhängigen Enzyms handelt oder Coenzym-abhängige Enzyme selbst. Bevorzugte Beispiele für Analyten sind Glucose, Milchsäure, Äpfelsäure, Glycerin, Alkohol, Cholesterin, Triglyceride, Ascorbinsäure, Cystein, Glutathion, Peptide, Harnstoff, Ammonium, Salicylat, Pyruvat, 5'-Nukleotidase, Creatinkinase (CK), Lactat-Dehydrogenase (LDH), Kohlendioxid etc. Bevorzugt ist der Analyt Glucose. Besonders bevorzugt ist hierbei der Nachweis von Glucose mit Hilfe von Glucose-Dehydrogenase (GlucDH).

Die Veränderung des stabilen Coenzyms durch Reaktion mit dem Analyten kann grundsätzlich auf beliebige Art und Weise nachgewiesen werden. Hier können grundsätzlich alle aus dem Stand der Technik bekannten Methoden zum Nachweis enzymatischer Reaktionen eingesetzt werden. Vorzugsweise wird jedoch die Veränderung des Coenzyms durch optische Methoden nachgewiesen. Optische Nachweismethoden umfassen beispielsweise die Messung von Absorption, Fluoreszenz, Circulardichroismus (CD), optische Rotationsdispersion (ORD), Refraktometrie etc.

Ein optisches Nachweisverfahren, welches im Rahmen der vorliegenden Anmeldung bevorzugt Anwendung findet, ist die Photometrie. Zur photometrischen Messung einer Veränderung des Coenzyms infolge Umsetzung mit dem Analyten bedarf es indessen der zusätzlichen Anwesenheit mindestens eines Mediators, welcher die Reaktivität des reduzierten Coenzyms erhöht und eine Übertragung von Elektronen auf einen geeigneten optischen Indikator oder ein optisches Indikatorsystem ermöglicht.

Als Mediatoren, welche für die Zwecke der vorliegenden Erfindung geeignet sind, kommen u.a. Nitrosoaniline, wie beispielsweise [(4-Nitrosophenyl)imino]dimethanol-Hydrochlorid, Chinone, wie beispielsweise Phenanthrenchinone, Phenanthrolinchinone oder Benzo[h]-chinolinchinone, Phenazine, wie beispielsweise 1-(3-Carboxypropoxy)-5-ethylphenaziniumtrifluormethansulfonat, oder/und Diaphorase (EC 1.6.99.2) in Frage. Bevorzugte Beispiele für Phenanthrolinchinone umfassen 1,10-Phenanthrolin-5,6-chinone, 1,7-Phenanthrolin-5,6-chinone, 4,7-Phenanthrolin-5,6-chinone sowie deren N-alkylierte oder N,N'-dialkylierte Salze, wobei im Falle N-alkylierter bzw. N,N'-dialkylierter Salze Halogenide, Trifluormethansulfonat oder andere die Löslichkeit erhöhende Anionen als Gegenion bevorzugt sind.

Als optischer Indikator oder als optisches Indikatorsystem kann eine beliebige Substanz verwendet werden, welche reduzierbar ist und bei Reduktion eine detektierbare Änderung ihrer optischen Eigenschaften, wie beispielsweise Farbe, Fluoreszenz, Remission, Transmission, Polarisation oder/und Brechungsindex, erfährt. Die Bestimmung des Vorhandenseins oder/und der Menge des Analyten in der Probe kann mit dem bloßen Auge oder/und mittels einer Detektionsvorrichtung unter Verwendung eines dem Fachmann geeignet erscheinenden photometrischen Verfahrens erfolgen. Vorzugsweise werden Heteropolysäuren, und insbesondere 2,18-Phosphormolybdänsäure, als optische Indikatoren verwendet, die zum entsprechenden Heteropolyblau reduziert werden.

Besonders bevorzugt wird die Veränderung des Coenzyms durch Messung der Fluoreszenz nachgewiesen. Die Fluoreszenzmessung ist hochsensitiv und ermöglicht den Nachweis selbst geringer Konzentrationen des Analyten in miniaturisierten Systemen.

Alternativ kann die Veränderung des Coenzyms auch elektrochemisch unter Verwendung eines geeigneten Testelements, wie beipielsweise eines elektrochemischen Teststreifens, nachgewiesen werden. Voraussetzung hierfür ist wiederum die Verwendung geeigneter Mediatoren, welche vom reduzierten Coenzym durch Übertragung von Elektronen in eine reduzierte Form überführt werden können. Die Bestimmung des Analyten erfolgt über eine Messung des zur Reoxidation des reduzierten Mediators benötigten Stromes, welcher mit der Konzentration des Analyten in der Probe korreliert. Beispiele für Mediatoren, welche für elektrochemische Messungen verwendet werden können, umfassen insbesondere die vorstehend erwähnten, für photometrische Messungen eingesetzten Mediatoren.

Zum Nachweis eines Analyten kann ein Flüssigtest verwendet werden, wobei das Reagenz z.B. in Form einer Lösung oder Suspension in einer wässrigen oder nichtwässrigen Flüssigkeit oder als Pulver oder Lyophilisat vorliegt. Es kann jedoch auch ein Trockentest verwendet werden, wobei das Reagenz auf einem Träger, einem Teststreifen, aufgebracht ist. Der Träger kann beispielsweise einen Teststreifen, umfassend ein saugfähiges oder/und quellbares Material, umfassen, das von der zu untersuchenden Probeflüssigkeit benetzt wird.

Ein besonders bevorzugtes Testformat, umfasst die Verwendung des Enzyms Glucose-Dehydrogenase mit einem stabilen NAD-Derivat zum Nachweis von Glucose, wobei ein Derivat des reduzierten Coenzyms NADH gebildet wird. Der Nachweis von NADH erfolgt durch optische Methoden, z.B. durch photometrische oder fluorometrische Bestimmung nach UV-Anregung. Ein besonders bevorzugtes Testsystem ist in US 2005/0214891 beschrieben, auf das hier ausdrücklich Bezug genommen wird.

Insbesondere kann die stabile Testchemie derart ausgestaltet sein, dass diese ein mit einem stabilen Coenzym stabilisiertes Enzym umfasst, wobei das stabilisierte Enzym bei einer Lagerung von vorzugsweise mindestens zwei Wochen, besonders bevorzugt mindestens vier Wochen und am meisten bevorzugt mindestens acht Wochen bei einer Temperatur von vorzugsweise mindestens 20 °C, besonders bevorzugt mindestens 25 °C und am meisten bevorzugt mindestens 30 °C, gegebenenfalls bei hoher Luftfeuchtigkeit und ohne Trockenreagenz eine Abnahme der enzymatischen Aktivität von weniger als 50 %, vorzugsweise weniger als 30 % und am meisten bevorzugt weniger als 20 % gegenüber dem Ausgangswert aufweist.

Auch andere Arten stabiler Testchemien können alternativ oder zusätzlich verwendet werden, beispielsweise die in WO 2007/012494 A1 beschriebene Testchemie. Die Testchemie kann grundsätzlich in beliebiger Weise in einem Testelement enthalten sein. Die Testchemie bzw. das Testelement können zur Durchführung von Trocken- oder Flüssigtests geeignet sein. Beispielsweise kann die Testchemie zu diesem Zweck auf einem geeigneten Trägermaterial aufgebracht sein, beispielsweise auf einem Kunststoff und/oder einem keramischen Material und/oder einem Papiermaterial.
Bei Verwendung einer zumindest weitgehend gegenüber Umwelteinflüssen stabilen Testchemie, kann aufbessere und präzisere Verbindungstechniken zur Herstellung des Gehäuses und/oder auf eine größere Materialauswahl zurückgegriffen werden. Dementsprechend wird in dem zweiten Aspekt der vorliegenden Erfindung vorgeschlagen, das analytische Magazin derart auszugestalten, dass dieses ein Gehäuse mit mindestens zwei Bauteilen aufweist, beispielsweise zwei Magazinhälften. Die beiden Teile müssen dabei nicht notwendigerweise gleich ausgestaltet sein. Das Gehäuse, insbesondere die mindestens zwei Teile gemeinsam, können die mindestens zwei Kammern bilden. Besonders bevorzugt ist es, wenn die mindestens zwei Teile miteinander durch ein Verfahren ohne Verwendung eines Klebemittels verbunden sind. Insbesondere bieten sich hier stoffschlüssige Verbindungsverfahren ohne Klebemittel an. Besonders bevorzugt aufgrund der hohen Präzision und der geringen Verursachung von Verunreinigungen ist die Verwendung mindestens eines Laserschweißverfahrens. Dementsprechend wird in dem zweiten Aspekt der Erfindung vorgeschlagen, die mindestens zwei Bauteile durch ein Laserschweißverfahren miteinander zu verbinden.
Mit einem Laserschweißverfahren lassen sich gleichmäßige Schweißnähte mit einer geringen Breite und hohen Präzision erzielen, wobei das Verfahren gleichzeitig thermisch gut kontrollierbar und lokalisierbar sowie praktisch verunreinigungsfrei durchführbar ist.
Insbesondere bei Verwendung eines Laserschweißverfahrens bietet es sich an, wie ebenfalls erfindungsgemäß vorgeschlagen wird, wenn ein erstes der mindestens zwei Bauteile und ein zweites der mindestens zwei Bauteile eine unterschiedliche Transparenz aufweisen. Beispielsweise kann ein erstes der mindestens zwei Teile nahezu vollständig transparent ausgestaltet und ein zweites der mindestens zwei Teile innerhalb des für das Laserschweißverfahren verwendeten Wellenlängenbereichs absorbierend ausgestaltet werden. Dabei können vorzugsweise die mindestens zwei Bauteile denselben Grundwerkstoff aufweisen, jedoch jeweils mit einer unterschiedlichen Absorption für Licht im sichtbaren und/oder infraroten und/oder ultravioletten Spektralbereich. Beispielsweise können die mindestens zwei Teile eine unterschiedliche Absorption in einem Spektralbereich zwischen 500 und 1200 nm aufweisen, insbesondere in einem Spektralbereich von 700 bis 1100 nm oder 700 bis 1000 nm, in welchem übliche, für das Laserschweißen verwendete Laser emittieren, beispielsweise Halbleiterlaser und/oder Nd:YAG-Laser. Um eine unterschiedliche Transparenz beziehungsweise Absorption der mindestens zwei Bauteile zu erreichen, kann ein Grundwerkstoff der Bauteile, welcher auch für beide Bauteile identisch sein kann, unterschiedlich angefärbt werden, beispielsweise indem der Grundwerkstoff (beispielsweise Polycarbonat, PC) eines der Teile mit einem Farbstoff versetzt wird, um eine Transparenz zu vermindern. Beispielsweise könnten die beiden Bauteile in diesem Wellenlängenbereich einen Transparenzunterschied von mindestens 5%, vorzugsweise von mindestens 20% und besonders bevorzugt von mindestens 50% für die verwendete Laserstrahlung aufweisen. Unter einer Transparenz wird dabei ein Transmissiongrad verstanden.

Wird ein Schweißverfahren zur Verbindung der mindestens zwei Teile verwendet, so können die Schweißnähte beispielsweise eine Breite von maximal 0,5 mm aufweisen, insbesondere von maximal 0,3 mm und besonders bevorzugt von maximal 0,2 mm. Dies trägt, wie unten noch ausgeführt wird, erheblich zur Erhöhung der Packungsdichte bei. Insbesondere macht sich hier wiederum vorteilhaft bemerkbar, dass die Testchemie zumindest weitgehend gegenüber Umwelteinflüssen stabilen ist, da in diesem Fall ein Feuchtigkeitsübertritt von einer Kammer in eine andere Kammer weit gehend irrelevant ist.

Allgemein lassen sich für das Gehäuse, insbesondere für die mindestens zwei Bauteile des Gehäuses, beispielsweise für die zwei Magazinhälften, eine Vielzahl von Materialien einsetzen. Insbesondere können thermoplastische Kunststoffe eingesetzt werden. Ein besonderer Vorteil der Verwendung einer gegenüber Umwelteinflüssen zumindest weitgehend stabilen Testchemie, besteht gerade darin, dass diese Kunststoffe keine besonderen Anforderungen hinsichtlich einer Undurchsichtigkeit gegenüber Feuchtigkeit erfüllen müssen. Dementsprechend kann die Auswahl und Gestaltung der Kunststoffe beispielsweise nach anderen Kriterien vorgenommen werden, beispielsweise nach einer Verarbeitbarkeit in einem speziellen Formgebungsprozess, beispielsweise beim Spritzgießen. Auch kann auf kostengünstige Werkstoffe zurückgegriffen werden. Beispielsweise lassen sich einer oder mehrere der folgenden Kunststoffe einsetzen: PC (Polycarbonat); ABS (Acrylnitril-Butadien-Styrol); COC (Cyclo-Olefin-Copolymere); PMMA (Polymethylmethacrylat); PS (Polystyrol); PET (Polyethylenterephthalat). Diese Materialien weisen hinsichtlich ihrer Verarbeitungseigenschaften und/oder hinsichtlich ihrer Kosten Vorteile auf, sind jedoch grundsätzlich nur schlecht verwendbar, wenn auch die Forderung nach einer Dampfdichtigkeit erfüllt sein muss.

PC weist beispielsweise eine hohe Beständigkeit gegenüber ionisierender Strahlung auf und eine hohe Transparenz für ein breites Spektrum. Es handelt sich um einen kostengünstigen Massenwerkstoff, welcher jedoch eine vergleichsweise hohe Durchlässigkeit für Wasserdampf aufweist. Da diese Durchlässigkeit jedoch grundsätzlich im Rahmen der vorliegenden Erfindung, insbesondere bei Verwendung der stabilen Testchemie, weit gehend irrelevant ist, und da die Verarbeitungseigenschaften dieses Werkstoffs in der Praxis besonders gut sind, ist dieser Werkstoff im Rahmen der vorliegenden Erfindung besonders bevorzugt.

ABS ist sehr gut zu verarbeiten und insbesondere sehr gut spritzgießbar, so dass auch die Verwendung dieses Werkstoffs vorteilhaft ist. Auch dieser Werkstoff weist eine vergleichsweise gute Transparenz für ein breites Lichtspektrum auf sowie geringe Kosten.

COC weist zwar eine hohe Transparenz in einem weiten Spektralbereich vom ultravioletten Licht bis hin zum infraroten Spektralbereich auf und stellt eine gute Dampfsperre bereit, ist jedoch vergleichsweise teuer und nur mäßig stabil gegenüber ionisierender Strahlung.

PMMA weist eine lediglich geringe oder gar keine Eigenfluoreszenz im ultravioletten Spektralbereich auf sowie eine gute Transparenz für ein breites Lichtspektrum. Die hohe Dampfdurchlässigkeit dieses Werkstoffs ist im Rahmen der vorliegenden Erfindung, insbesondere bei Verwendung der stabilen Testchemie, tolerierbar, und es handelt sich um einen kostengünstigen Werkstoff. Auch dieses Material kann daher im Rahmen der vorliegenden Erfindung vorteilhaft eingesetzt werden.

PS ist gut zu verarbeiten, insbesondere durch ein Spritzgießverfahren. Es weist eine gute Transparenz für ein breites Lichtspektrum auf. Zudem handelt es sich um einen kostengünstigen Massenkunststoff. Insgesamt ist also auch dieses Material im Rahmen der vorliegenden Erfindung gut einsetzbar.

Mit dieser erweiterten und nicht mehr durch die Forderung nach Dampfundurchlässigkeit eingeschränkten Materialauswahl ist es grundsätzlich möglich, die vorzugsweise mindestens zwei Teile des Gehäuses des analytischen Magazins mittels Laserschweißens zu verbinden, anstelle eines herkömmlichen eines Ultraschallschweißens und/oder eines Klebeverfahrens, welches für undurchsichtige Werkstoffe übrig bliebe. Gleiche Werkstoffe lassen sich beispielsweise mittels eines Lasers sehr einfach verschweißen, beispielsweise PC mit PC und/oder COC mit COC usw., insbesondere wenn ein Teil lichtabsorbierend für die Laserwellenlänge ist, beispielsweise durch eine entsprechende Anfärbung und/oder Dotierung, und wobei das andere Teil transparent oder transparenter ausgestaltet ist. Zwar lassen sich grundsätzlich auch opake Teile so durchstrahlen, dass eine Schweißung möglich ist, aber die Schweißnähte werden dann in der Regel gröber und brauchen mehr Platz. Bei hoher Transparenz und glatten Oberflächen der miteinander zu verbindenden Teile lassen sich hingegen sehr schmale Schweißnähte erzielen, beispielsweise Schweißnähte mit der oben genannten Breite von 0,3 mm. Diese geringen Schweißnähte erlauben es, das analytische Magazin sehr klein zu gestalten, beispielsweise mit den oben beschriebenen bevorzugten Packungsdichten. Weiterhin lässt sich beim Laserschweißen die Bildung von Staub vermeiden, welche üblicherweise bei anderen Schweißverfahren auftritt, beispielsweise beim Ultraschallschweißen. Eine derartige Bildung von Staub kann sich insbesondere bei analytischen Hilfsmitteln oder Teil-Hilfsmitteln in Form von Testelementen negativ bemerkbar machen, da eine Testchemie der Testelemente durch Staub und sonstige Verunreinigungen, die beim Schweißen auftreten können, kontaminiert werden kann. Werden Lanzetten und/oder Microsampler als analytische Hilfsmittel oder als analytische Teil-Hilfsmittel eingesetzt, so macht das ebenfalls eine Staubbildung sehr negativ bemerkbar, da beispielsweise eine Hydrophilie der Lanzetten und/oder Microsampler durch den Staub beeinflusst werden kann. Dies kann durch die Verwendung des Laserschweißverfahrens vermieden werden. Weiterhin treten keine Vibrationen auf, welche Strukturen oder Teile des analytischen Magazins in einer Resonanz versetzen könnten. Werden auch keine Zusatzwerkstoffe nötig, wie beispielsweise Klebstoffe, welche das Innere der Kammern und/oder die analytischen Hilfsmitteln verunreinigen könnten, so dass beispielsweise eine Hydrophilie von Lanzetten oder Microsamplern gefährdet wäre.
Auch werden insbesondere durch Verwendung des Laserschweißens und/oder der bevorzugten Kunststoffe, neue Verfahren zum Verschließen und/oder Versiegeln des analytischen Magazins, beispielsweise zur Versiegelung eines fertigen analytischen Magazins, möglich. Bislang werden in vielen Fällen analytische Magazine mit heißklebefähigen Folien thermisch verschlossen. Die dabei verwendeten Heißkleber können jedoch unter Umständen die analytischen Hilfsmittel beeinflussen. So können beispielsweise Dämpfe des Heißklebers Lanzetten und/oder Microsampler beeinflussen, beispielsweise deren Hydrophilie beeinträchtigen. Mittels der vorgeschlagenen Erfindung, insbesondere unter Verwendung des Laserschweißverfahrens und/oder der vorgeschlagenen Materialien, ist es jedoch möglich, alternativ oder zusätzlich zu beispielsweise Metallfolien, z.B. Aluminiumfolien, zur Versiegelung des analytischen Magazins eine oder mehrere Kunststofffolien zu verwenden, welche beispielsweise mittels eines Laser angeschweißt statt angeklebt werden können.

Insgesamt bietet die neu gewonnene Freiheit hinsichtlich der Auswahl der Materialien somit eine Grundlage für ein erheblich kompakteres System. Dies ist nicht nur dadurch bedingt, dass das analytische Magazin mit einem erheblich geringeren Bauraum und/oder einer erheblich höheren Packungsdichte ausgestaltet werden kann. Das analytische Magazin kann auch in seiner Herstellung und/oder seiner Handhabung stark vereinfacht und kostengünstiger ausgestaltet werden.

Das analytische Magazin weist allgemein, vorzugsweise eine oder mehrere der folgenden Eigenschaften auf: ein Gesamtvolumen von nicht mehr als 10 cm³; einen Außenradius von nicht mehr als 5 cm; einen Innenradius zwischen 0,5 cm und 2 cm; eine Dicke von nicht mehr als 1 cm; eine Anzahl an analytischen Hilfsmitteln von 10 bis 100; ein Volumen zwischen 3 cm³ und 30 cm³; eine Packungsdichte der analytischen Hilfsmittel von mehr als 5/cm³.
Besonders bevorzugt ist es, wenn ein Außenvolumen des analytischen Magazins, also ein Volumen ohne Berücksichtigung von Löchern oder anderen Öffnungen in dem analytischen Magazin, 5 cm³ nicht überschreitet, vorzugsweise 3 cm³ und besonders bevorzugt 2 cm³. Beispielsweise kann das Außenvolumen 1,94 cm³ betragen. Besonders bevorzugt ist es, wenn ein Leervolumen des analytischen Magazins, also ein gesamtes Volumen von optional vorhandenen Öffnungen in dem analytischen Magazin, 0,8 cm³ nicht überschreitet, vorzugsweise 0,5 cm³ und besonders bevorzugt 0,4 cm³. Beispielsweise kann das Leervolumen 0,39 cm³ betragen. Bei den Öffnungen kann es sich beispielsweise um innere Öffnungen eines scheibenförmigen analytischen Magazins handeln, in welche beispielsweise ein Antrieb eingreifen kann. Nicht umfasst von diesem Leervolumen soll der Innenraum im Inneren des Gehäuses sein, beispielsweise die Kammern. Unter einem Nettovolumen wird dabei im Rahmen der vorliegenden Erfindung allgemein das Außenvolumen abzüglich des Leervolumens verstanden. Bei einem kreisscheibenförmigen Magazin ergibt sich somit ein Volumen, das sich im Wesentlichen aus dem Durchmesser und der Höhe der Kreisscheibe ergibt, und bei einem ringscheibenförmigen Magazin ein Nettovolumen, das sich gegenüber dem Nettovolumen einer entsprechenden Kreisscheibe durch Abzug des Volumens einer zentralen Ausnehmung ergibt. Dementsprechend ist es bevorzugt, wenn das Nettovolumen des analytischen Magazins, also das Außenvolumen abzüglich des Leervolumens, 5 cm³ nicht überschreitet, besonders bevorzugt 3 cm³ und insbesondere 2 cm³ nicht überschreitet. Beispielsweise kann das Nettovolumen 1,55 cm³ betragen.

Unter einer Packungsdichte wird allgemein im Rahmen der vorliegenden Erfindung eine Anzahl analytische Hilfsmittel pro Nettovolumen des analytischen Magazins verstanden. Wie oben dargelegt, kann jedes analytische Hilfsmittel jedoch mehrere Teil-Hilfsmittel umfassen, welche miteinander zusammenwirken können, beispielsweise jeweils als ein Test. Vorzugsweise umfasst jedes analytische Hilfsmittel als Teil-Hilfsmittel ein Testelement mit mindestens einer Testchemie. Zusätzlich kann dann jedes analytische Hilfsmittel als weiteres Teil-Hilfsmittel mindestens eine Lanzette umfassen, welche zur Probengenerierung einer Probe an Körperflüssigkeit dient, die dann auf das zugehörige Testelement aufgebracht wird. Sind in einem analytischen Hilfsmittel mehrere analytische Teil-Hilfsmittel enthalten, so zählen zusammengehörige analytische Teil-Hilfsmittel für die Berechnung der Packungsdichte jedoch nach wie vor als ein analytisches Hilfsmittel, beispielsweise indem ein Testelement und eine zugehörige Lanzette als ein gemeinsames analytisches Hilfsmittel gezählt werden. Beispielsweise kann jeweils genau ein analytisches Hilfsmittel mit mindestens einem Testelement und optional mindestens einer Lanzette in jeweils einer Kammer aufgenommen sein. Wie oben dargelegt, sind jedoch auch andere Ausgestaltungen möglich.

So kann das analytische Magazin beispielsweise als Kreisringscheibe ausgestaltet sein, mit einem Außendurchmesser von weniger als 100 mm, beispielsweise 50 mm, und einem Innendurchmesser einer Ausnehmung der Kreisringscheibe von weniger als 50 mm, beispielsweise 22,5 mm. Das analytische Magazin kann beispielsweise eine Dicke von weniger als 5,0 mm aufweisen, beispielsweise eine Dicke von 3,1 mm. Das analytische Magazin kann vorzugsweise mehr als 20 analytische Hilfsmittel umfassen, beispielsweise mindestens 50 analytische Hilfsmittel und sogar 100 analytische Hilfsmittel oder mehr. Beispielsweise können 50 analytische Hilfsmittel mit jeweils einem Testelement und optional jeweils zusätzlich einer Lanzette vorgesehen sein, wobei jeweils ein Testelement und eine zugehörige Lanzette als ein analytisches Hilfsmittel, auch "Test" genannt, zählen. Beispielsweise kann jeweils ein derartiges analytisches Hilfsmittel in jeweils einer Kammer aufgenommen sein. Dementsprechend kann die Packungsdichte beispielsweise mindestens 50 analytische Hilfsmittel / 5 cm³ = 1 analytisches Hilfsmittel / 0,1 cm³ betragen, vorzugsweise mindestens 50 analytische Hilfsmittel / 3 cm³ = 1 analytisches Hilfsmittel / 0,06 cm³ und besonders bevorzugt mindestens 50 analytische Hilfsmittel / 2 cm³ = 1 analytisches Hilfsmittel / 0,04 cm³. Beispielsweise kann die Packungsdichte bei 50 analytische Hilfsmittel / 1,55 cm³ = 1 analytisches Hilfsmittel / 0,031 cm³ liegen. Auch eine andere Ausgestaltung des analytischen Magazins als eine Ausgestaltung als Kreisscheibe ist jedoch möglich, beispielsweise in einer oder mehreren der oben beschriebenen Geometrien. Auch eine Ausgestaltung als Bandmagazin ist beispielsweise möglich, wobei beispielsweise eine Kammer des Bandmagazins einen Gutwickel mit noch nicht benutzten analytischen Hilfsmitteln umfasst und eine andere Kammer des Bandmagazins einen Schlechtwickel, auf welchem bereits benutzte analytische Hilfsmittel aufnehmbar sind. Hohe Packungsdichten lassen sich insbesondere auch dadurch erreichen, dass die analytischen Hilfsmittel sehr dicht beieinander gelagert werden, ohne dass eine hermetische Trennung zwischen den analytischen Hilfsmitteln erfolgen muss. Diesbezüglich macht sich die Verwendung der stabilen Testchemie besonders vorteilhaft bemerkbar. So können beispielsweise analytische Magazine verwendet werden, welche ein Gehäuse aufweisen, welches eine Wandstärke von maximal 1,2 mm aufweist. Unter einer Wandstärke ist dabei eine Dicke des Gehäuses zwischen einer ein analytisches Hilfsmittel umfassenden Kammer und der Umgebung oder einer benachbarten Kammer zu verstehen, insbesondere an einer dünnsten Stelle des Gehäuses. Besonders bevorzugt ist es, wenn die Wandstärke nicht mehr als 1,0 mm und insbesondere nicht mehr als 0,8 mm beträgt. Beispielsweise kann die Wandstärke von 0,3 mm bis 0,8 mm gewählt werden.
Wiederum alternativ oder zusätzlich kann in dem analytischen Magazin auf die Verwendung eines Trockenmittels wie beispielsweise Aktivkohle verzichtet werden. Dementsprechend ist es besonders bevorzugt, wenn das analytische Magazin Trockenmittels-frei ausgestaltet ist. Auch auf diese Weise lässt sich Bauraum einsparen.
Wenn eine derartig stabile Testchemie verwendet wird, so kann grundsätzlich auf eine feuchtigkeitsundurchlässige Ausgestaltung der Trennung der einzelnen Kammern auch vollständig verzichtet werden. Das analytische Magazin kann also allgemein eine Mehrzahl von analytischen Hilfsmitteln, welche in mindestens zwei Kammern aufgenommen sind, umfassen. Die analytischen Hilfsmittel können dabei wiederum jeweils mindestens ein Testelement mit mindestens einer Testchemie zum Nachweis mindestens eines Analyten in einer flüssigen Probe, insbesondere einer Körperflüssigkeit umfassen, wobei die Testchemie derart ausgestaltet ist, dass diese zumindest weitgehend stabil gegenüber Umwelteinflüssen, insbesondere gegenüber Feuchtigkeit, ist. Dabei können allgemein die Kammern derart voneinander getrennt ausgestaltet sein, dass ein Austausch von Feuchtigkeit zwischen den Kammern, beispielsweise zwischen benachbarten Kammern, möglich ist. Beispielsweise können die Kammern Kammerwände aufweisen, wobei in oder neben den Kammerwänden Spalte oder andere Öffnungen vorgesehen sind, vorzugsweise mit einer Öffnungsweite von nicht mehr als 20 µm, insbesondere von nicht mehr als 10 µm. Diese Öffnungsweiten ermöglichen einerseits einen Austausch von Luftfeuchtigkeit zwischen den Kammern, halten jedoch in der Regel gröbere Verunreinigungen oder Keime zurück.

Das analytische Magazin in einer oder mehreren der oben beschriebenen Ausgestaltungen lässt sich zusätzlich auf verschiedene Weisen vorteilhaft weiter ausgestalten. Insbesondere kann, wie oben dargestellt, ein analytisches Hilfsmittel jeweils ein Testelement mit einer Testchemie, insbesondere der gegenüber Umwelteinflüssen stabilen Testchemie, und eine Lanzette umfassen. Diese Teil-Hilfsmittel können gemeinsam in ein und derselben Kammer gelagert sein, beispielsweise jeweils ein Paar aus einer Lanzette und einem Testelement in einer Kammer, oder mehrere derartiger Paare in einer gemeinsamen Kammer. Durch Verwendung der gegenüber Umwelteinflüssen, insbesondere Luftfeuchtigkeit und/oder Betastrahlung stabilen Testchemie, kann eine primäre Forderung nach einer separaten Verpackung von Lanzetten und Testelementen entfallen. Darüber hinaus kann auch die Notwendigkeit, die Testchemie wasserdampfdicht zu verpacken und/oder ein Trockenmittel in die Kammern und/oder das analytische Magazin einzufügen, entfallen. Dadurch werden neue Konzepte insbesondere für ein kombiniertes analytisches Magazin mit Lanzetten und Testelementen möglich. Eine voneinander getrennte Aufbewahrung der Lanzetten und der Testelemente ist nicht erforderlich. Zudem kommen, wie unten noch näher ausgeführt wird, andere Materialien für die Magazinteile infrage, da nicht mehr gleichzeitig die Forderungen nach Strahlenstabilität und Dampfdichtigkeit zusammen erfüllt werden müssen.

Da auf eine Barriere zwischen den analytischen Hilfsmitteln, insbesondere zwischen Lanzetten und Testelementen, verzichtet werden kann, kann eine erhebliche kompaktere Anordnung von Lanzettenelementen und Testelementen erfolgen, beispielsweise in derselben Kammer des analytischen Magazins. Die Lanzettenspitze kann beispielsweise bei der Lagerung in der Kammer dicht neben der Testchemie liegen, die sich nach einer Probengewinnung auch als Position für einen Übertrag der Probe auf die Testchemie eignet. Dadurch kann ein Mechanismus zur Betätigung des analytischen Hilfsmittels erheblich einfacher gestaltet werden als bei üblichen analytischen Magazinen, da beispielsweise auf eine zusätzliche Bewegung zum Zweck einer Beseitigung einer zweiten Barriere, als einer Barriere zwischen Lanzette und Testelement, verzichtet werden kann und da keine zusätzliche Position mit der Lanzette angefahren werden muss. Im Unterschied zu bisherigen Konzepten mit getrennter Verpackung von Lanzette und Testchemie muss die Testchemie auch nicht notwendigerweise bewegt werden, um diese aus ihrer Verpackung zu entfernen. In diesem Fall muss in der Regel lediglich noch die Lanzette an einen entsprechenden Aktor angedockt und bewegt werden.

Dementsprechend wird weiterhin in einem zusätzlichen Aspekt der vorliegenden Erfindung ein weiteres Verfahren zur Herstellung eines analytischen Magazins vorgeschlagen. Bei dem analytischen Magazin kann sich insbesondere um ein analytisches Magazin gemäß einer oder mehrerer der oben beschriebenen Ausgestaltungen handeln und/oder ein analytisches Magazin, welches gemäß einem Verfahren gemäß einer oder mehreren der oben beschriebenen Ausgestaltungen herstellbar ist. Auch andere Ausgestaltungen sind jedoch grundsätzlich möglich.

Das analytische Magazin ist eingerichtet, um eine Mehrzahl von analytischen Hilfsmitteln in einer Mehrzahl von Kammern aufzunehmen. Die analytischen Hilfsmittel weisen jeweils mindestens eine Testchemie auf. Insbesondere kann es sich dabei um eine gegenüber Umwelteinflüssen zumindest weit gehend stabile Testchemie gemäß der obigen Beschreibung handeln. Die Testchemie kann beispielsweise Bestandteil von Testelementen sein, welche vorzugsweise vollständig in dem Gehäuse aufgenommen sind, beispielsweise in den Kammern. Bei dem erfindungsgemäßen Verfahren wird eine Mehrzahl analytischer Hilfsmittel in mindestens eine der Kammern eingebracht. Beispielsweise kann genau ein analytisches Hilfsmittel pro Kammer eingebracht werden, oder es können eine Mehrzahl analytischer Hilfsmittel in eine Kammer eingebracht werden. Das analytische Magazin weist weiterhin ein Gehäuse mit mindestens zwei Bauteilen auf. Die mindestens zwei Bauteile werden vor oder nach dem Einbringen der analytischen Hilfsmittel in die mindestens eine Kammer miteinander durch ein Laserschweißverfahren verbunden. Die analytischen Hilfsmittel können zusätzlich jeweils mindestens eine Lanzette aufweisen. Bevorzugt weisen diese Lanzetten mindestens einen Kapillarkanal zur Aufnahme von Körperflüssigkeit auf, welche zu dem Testelement über die Lanzette geleitet wird. Vorteilhafterweise ist ein derartiger Kapillarkanal der Lanzette (beispielsweise eines Nadelelements), insbesondere eine Kapillarstruktur, beschichtet, vorzugsweise hydrophil beschichtet um einen verbesserten Transport der Körperflüssigkeit zu ermöglichen. Das analytische Magazin kann weiterhin insbesondere nach Durchführung des Laserschweißverfahrens mit ionisierender Strahlung sterilisiert werden. Für die Vorteile des vorgeschlagenen Verfahrens in einer oder mehreren der beschriebenen Ausgestaltungen kann weit gehend auf die obige Beschreibung verwiesen werden. Insbesondere bewirkt das Laserschweißverfahren, dass eine hohe Packungsdichte realisiert werden kann und dass die analytischen Hilfsmittel, insbesondere die Lanzetten, während des Herstellungsverfahrens nicht oder nur unwesentlich kontaminiert werden, beispielsweise mit Staub, der sich auf der hydrophilen Beschichtung der Lanzette ablagern könnte.

### Kurze Beschreibung der Figuren

Weitere Einzelheiten und Merkmale der Erfindung ergeben sich aus der nachfolgenden Beschreibung von bevorzugten Ausführungsbeispielen, insbesondere in Verbindung mit den Unteransprüchen. Hierbei können die jeweiligen Merkmale für sich alleine oder zu mehreren in Kombination miteinander verwirklicht sein. Die Erfindung ist nicht auf die Ausführungsbeispiele beschränkt. Die Ausführungsbeispiele sind in den Figuren schematisch dargestellt. Gleiche Bezugsziffern in den einzelnen Figuren bezeichnen dabei gleiche oder funktionsgleiche bzw. hinsichtlich ihrer Funktion einander entsprechende Elemente.

Im Einzelnen zeigen:
- Figuren 1A und 1B: verschiedene perspektivische Darstellungen eines ersten Bauteils eines analytischen Magazins;
- Figuren 2A und 2B: eine Bereitstellung einer Mehrzahl analytischer Hilfselemente in Form von Lanzetten;
- Figur 3: das erste Bauteil gemäß den Figuren 1A und 1B nach Einfügen der Lanzetten gemäß den Figuren 2A und 2B;
- Figur 4: eine perspektivische Darstellung eines zweiten Bauteils des analytischen Magazins;
- Figur 5: eine perspektivische Darstellung des analytischen Magazins nach Aufbringen des zweiten Bauteils gemäß Figur 4;
- Figur 6: eine exemplarische Darstellung eines Versiegelungselements zum Versiegeln von Öffnungen des analytischen Magazins; und
- Figuren 7A und 7B: verschiedene perspektivische Darstellungen eines fertigen analytischen Magazins.

### Ausführungsbeispiele

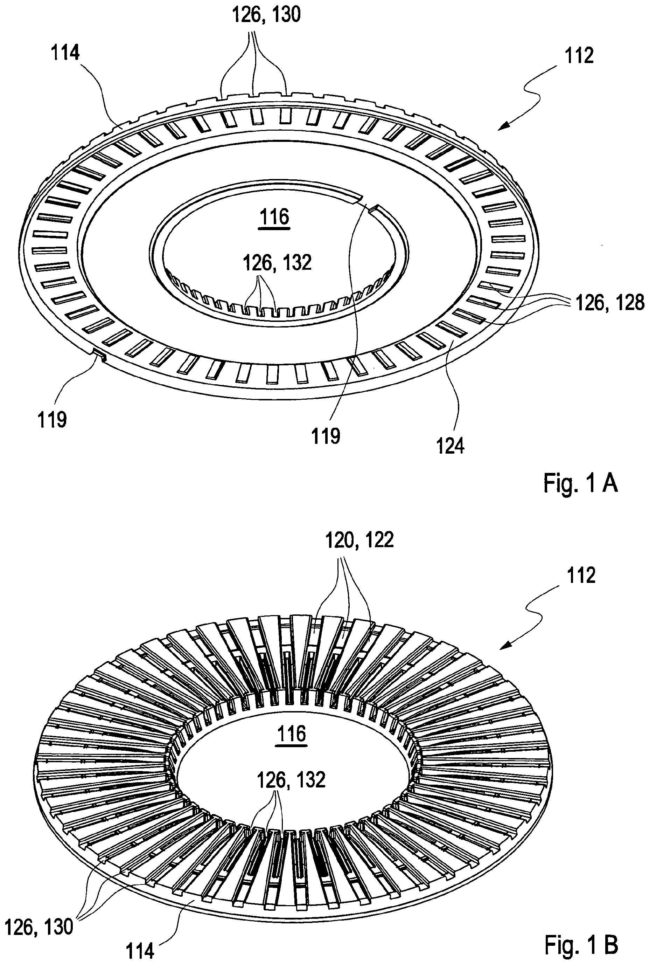

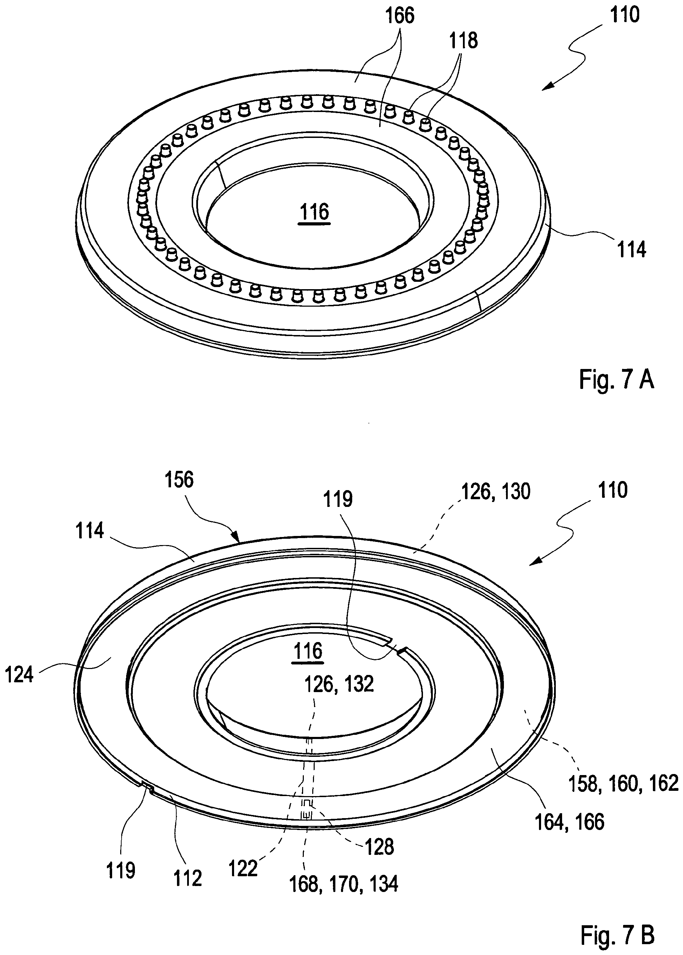

Anhand der Figuren 1A - 7B werden im Folgenden eine mögliche Ausführungsform eines erfindungsgemäßen Verfahrens zur Herstellung eines analytischen Magazins 110 beschrieben. Das fertige analytische Magazin 110 ist in den Figuren 7A und 7B dargestellt. Das analytische Magazin 110 stellt dabei ein Ausführungsbeispiel des oben beschriebenen ersten Aspekts der Erfindung dar, bei welchem bei der Herstellung mehrere analytische Hilfsmittel gleichzeitig in ein Gehäuse eingebracht werden. Das analytische Magazin 110 kann jedoch auch als Ausführungsbeispiel für den oben beschriebenen zweiten Aspekt der Erfindung fungieren, nach welchem ein Laserschweißverfahren als Verbindungstechnik eingesetzt wird.

In einem ersten Verfahrensschritt, welcher in den Figuren 1A und 1B dargestellt ist, wird ein erstes Bauteil 112 des analytischen Magazins 110 bereitgestellt. Dabei zeigt Figur 1A eine Ansicht des ersten Bauteils 112 von unten, also eine perspektivische Darstellung schräg von einer dem Innenraum des analytischen Magazins 110 abweisenden Seite des ersten Bauteils 112, wohingegen Figur 1B eine perspektivische Darstellung schräg von oben zeigt, also von einer Seite her, welche im zusammengebauten Zustand des analytischen Magazins 110 dem Inneren des analytischen Magazins 110 zuweist.

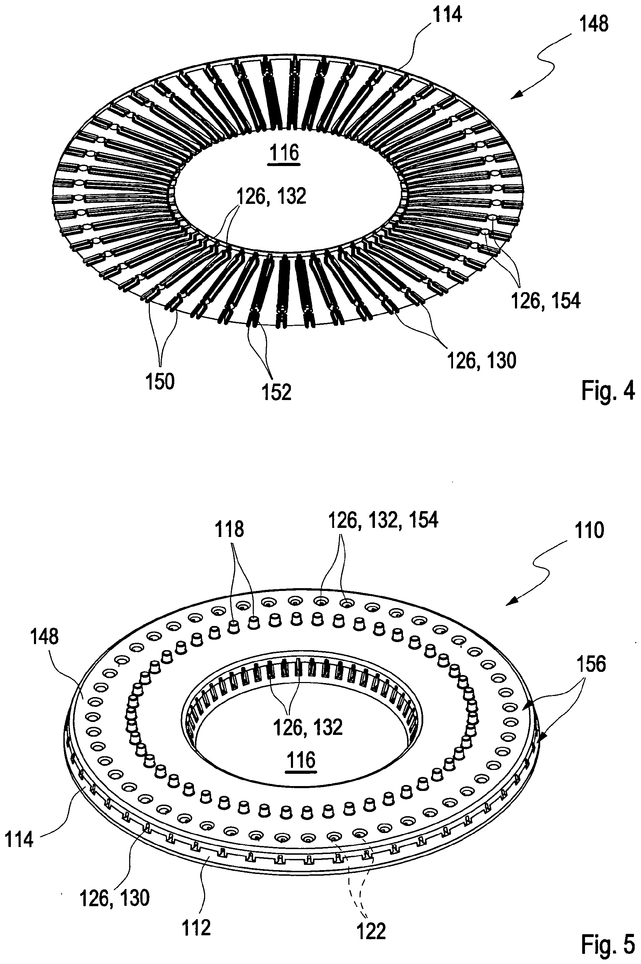

Wie aus den Darstellungen erkennbar ist, ist das erste Bauteil 112, wie auch das analytische Magazin 110 als Ganzes, in Form einer Kreisringscheibe ausgestaltet, mit einer äußeren Umfangsseite 114 und einer kreisförmigen Innenöffnung 116. Ein das analytische Magazin 110 verwendendes analytisches System, welches in den Figuren nicht dargestellt ist, kann beispielsweise das analytische Magazin 110 vollständig oder teilweise aufnehmen und kann vollständig oder teilweise in die Innenöffnung 116 eingreifen. So kann beispielsweise ein Aktor und/oder eine Zentrierung des analytischen Systems vollständig oder teilweise in die Verzahnung der Innenöffnung 116 eingreifen. Das analytische System kann beispielsweise über eine entsprechende Transportvorrichtung verfügen, welche mit Transportelementen 118 (siehe beispielsweise Figur 5 unten) an dem analytischen Magazin 110 zusammenwirkt, beispielsweise um einen Weitertransport beispielsweise ein Weitertakten, des analytischen Magazins 110 zu bewirken. Diesbezüglich kann beispielsweise auf den Stand der Technik verwiesen werden. Die Transportelemente können beispielsweise entsprechende Nuten, Zähne, Haken, Zapfen, Vorsprünge, Vertiefungen oder ähnliches umfassen. Die Transportelemente 118 sind beispielsweise in der unten näher beschriebenen Figur 5 dargestellt und dort exemplarisch zapfenförmig ausgestaltet. Weiterhin umfasst das analytische Magazin 110 optional in Figur 1A Einkerbungen 119. Diese Einkerbungen 119 können beispielsweise zur Positionierung in einer Montagevorrichtung dienen, wie im dargestellten Beispiel die äußere der Einkerbungen 119. Die innere Einkerbung 119 dient im dargestellten Beispiel der Versenkung eines Angusses bei einer Herstellung durch ein Formgebungsverfahren, beispielsweise ein Spritzgießverfahren.

Das erste Bauteil 112 kann, wie in den Figuren 1A und 1B dargestellt, somit beispielsweise als kreisringförmige Scheibe ausgestaltet sein und kann beispielsweise vollständig oder teilweise aus einem Kunststoffmaterial hergestellt sein. Das erste Bauteil 112 weist eine Mehrzahl von Aufnahmen 120 auf, welche im zusammengebauten Zustand des analytischen Magazins 110 Bestandteile von Kammern 122 bilden. Diese Aufnahmen 120 bzw. Kammern 122 sind in Figur 1B erkennbar. Wie aus dieser Darstellung ersichtlich, sind die Aufnahmen 120 im dargestellten Ausführungsbeispiel radial angeordnet und weisen entsprechende Vertiefungen im ersten Bauteil 112 auf. Die Aufnahmen 120 sind vorzugsweise gerade so breit, dass ein im Nachfolgenden näher beschriebenes Stech- und Sammelelement, als analytisches Hilfsmittel und/oder Teil-Hilfsmittel, gerade in diesen Aufnahmen 120 gelagert werden kann. Dementsprechend können beispielsweise die äußeren Abmessungen der Aufnahme 120 diesen analytischen Hilfsmitteln entsprechen, zuzüglich ggf. eines Spielraums, beispielsweise jeweils weniger hundertstel Millimeter in einer oder mehreren Dimensionen, in Form beispielsweise eines Spalts, was die Beweglichkeit der Stech- und Sammelelemente bzw. analytischen Hilfsmittel gewährleisten kann.

Auf der gegenüberliegenden Seite, also auf der von den Aufnahmen 120 wegweisenden Seite, weist das erste Bauteil 112 im dargestellten Ausführungsbeispiel eine Ringnut 124 auf Innerhalb dieser Ringnut 124 sind im dargestellten Ausführungsbeispiel jeweils Öffnungen 126 vorgesehen, wobei im dargestellten Ausführungsbeispiel jeweils eine dieser Öffnungen 126 pro Kammer 122 vorgesehen ist. Diese Öffnungen 126 sind im dargestellten Ausführungsbeispiel in Form länglicher, radial verlaufender Schlitze ausgebildet. Diese Öffnungen 126 dienen im dargestellten Ausführungsbeispiel später als Testelementöffnungen 128 oder Testfeldfenster, welche jeweils ein von den Kammern 122 aus zugängliches Testfeld definieren. Dies wird unten näher erläutert.

Neben den Testelementöffnungen 128 weisen die Kammern 122 im dargestellten Ausführungsbeispiel weitere Öffnungen 126 auf. Diese Öffnungen 126 sind teilweise bereits im ersten Bauteil 112 ausgebildet, können jedoch auch ganz oder teilweise in weiteren Bauteilen des analytischen Magazins 110 aufgenommen sein. So weisen die Aufnahmen 120 im dargestellten Ausführungsbeispiel, wie insbesondere aus Figur 1B ersichtlich ist, an der äußeren Umfangsseite 114 Öffnungen 126 auf, welche später als Probennahmeöffnungen 130 dienen. Durch diese Probennahmeöffnungen 130 können die analytischen Hilfsmittel vollständig oder teilweise für eine Probennahmebewegung austreten. Weiterhin weisen die Aufnahmen 120 an der der Innenöffnung 116 zuweisenden Seite Öffnungen 126 auf, welche im späteren Betrieb des analytischen Magazins 110 als Aktoröffnungen 132 fungieren und einen Eintritt eines nicht dargestellten Aktors ins Innere der Kammern 122, beispielsweise in einer gerade in einer Applikationsposition befindlichen Kammer 122 des analytischen Magazins 110, ermöglichen.

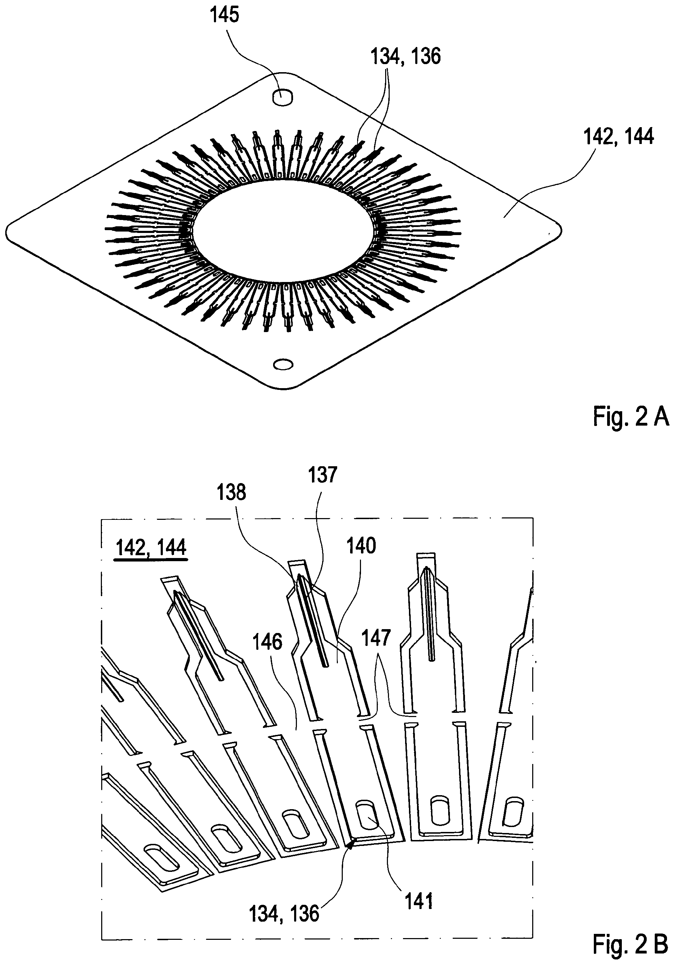

In einem weiteren, in den Figuren 2A und 2B dargestellten Verfahrensschritt, wird eine Mehrzahl analytischer Hilfsmittel 134 bereitgestellt. Im dargestellten Fall handelt es sich um Lanzetten 136 in Form von Microsamplern, welche jeweils ein analytisches Hilfsmittel 134 bilden können oder ein Teil-Hilfsmittel dieser analytischen Hilfsmittel 134. Dabei zeigt Figur 2A eine perspektivische Gesamtdarstellung der bereitgestellten Lanzetten 136, wohingegen Figur 2B eine Detaildarstellung zeigt. Die Lanzetten 136 sind im dargestellten Ausgangsbeispiel als Microsampler ausgestaltet und weisen ein verjüngtes, nach außen weisendes Lanzettenende 138 mit einer Lanzettenspitze auf, sowie jeweils einen verbreiteten Lanzettenkörper 140. An dem der Lanzettenspitze gegenüberliegenden Ende können die Lanzetten 136 ein oder mehrere Koppelelemente 141 zum Ankoppeln eines Aktors umfassen, beispielsweise eine Öse, ein Pilotloch oder ähnliches. Jede Lanzette 136 weist einen Kapillarkanal 137, beispielsweise einen Kapillarspalt, auf, welcher in Figur 2B durch eine gestrichelte Linie angedeutet ist und welcher der Aufnahme einer Blutprobe dient. Die Lanzetten 136 können beispielsweise als Flachlanzetten aus einer in Figur 2A erkennbaren Metallscheibe 142 herausgearbeitet sein, beispielsweise herausgeätzt sein. Die Metallscheibe 142 kann beispielsweise eine einzige radiale Lanzettenanordnung tragen oder mehrere Lanzettenanordnungen, welche beispielsweise nacheinander in verschiedene Magazingehäuse appliziert werden können.

Die Metallscheibe 142 und/oder Teile dieser Metallscheibe 142 dienen als Halteelement 144, mittels dessen die Lanzetten 136 untereinander verbunden sind. Dieses Halteelement 144 kann beispielsweise ein Ätzgitter umfassen, welches aus der Metallscheibe 142 herausgeätzt wurde. Die Lanzetten 136 können mit dem Halteelement 144 durch Verbindungselemente 146 verbunden sein, welche Bestandteil des Halteelements 144 sein können oder welche auch unmittelbar das Halteelement 144 bilden können, beispielsweise indem die Lanzetten 136 untereinander direkt über Stege oder ähnliches verbunden sind. Die Verbindungselemente 146 können beispielsweise in in Figur 2B erkennbaren Verjüngungen 147 im Lanzettenkörper 140 angreifen. Die Verbindungselemente können insbesondere Sollbruchstellen umfassen, welche ein leichteres Herausbrechen der Lanzetten 136 aus dem in den Figuren 2a und 2B dargestellten Verbund ermöglichen. Durch die Verjüngungen 147 wird gewährleistet, dass Bruchgrate vom Rand des Lanzettenkörpers 140 nach innen versetzt sind, so dass durch diese Bruchreste eine Bewegung der Lanzetten 136 in den Kammern 122 nicht behindert wird.

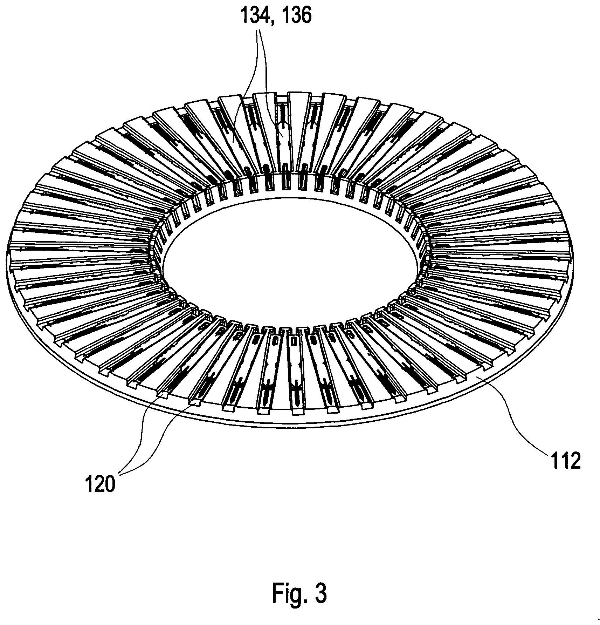

Die Lanzetten 136 sind mittels des Halteelements 144 bei der Bereitstellung in den Figuren 2A und 2B in radialer Ausrichtung bereitgestellt, so dass jeweils eine Lanzette 136 entsprechend der Aufnahmen 120 in Figur 1B orientiert ist. Anschließend werden die derart miteinander verbundenen analytischen Hilfsmittel 134 bzw. Lanzetten 136, optional von dem Halteelement 144 getrennt, beispielsweise durch Herausbrechen, und in die Aufnahmen 120 des ersten Bauteils 112 eingelegt und von dem Halteelement 144 getrennt, beispielsweise durch Herausbrechen. Das Herausbrechen kann vor und/oder nach dem Einlegen in die Aufnahmen 120 erfolgen. Das Ergebnis dieser Verfahrensschritte ist in Figur 3 dargestellt. Die Darstellung erfolgt hier analog zur Figur 1B, so dass weitgehend auf diese Figur verwiesen werden kann.

Das Einlegen der analytischen Hilfsmittel 134 bzw. Lanzetten 136 in die Aufnahmen 120 kann beispielsweise dadurch erfolgen, dass das Halteelement 144 mittels eines Saugers, eines Greifers oder ähnlicher Vorrichtungen ergriffen und entsprechend positioniert wird. Dieser Vorgang kann automatisch oder auch manuell erfolgen. Die Metallscheibe 142 und/oder das Halteelement 144 können zu diesem Zweck weitere Positionierungshilfen umfassen, beispielsweise die in Figur 2A angedeuteten Positionierungsöffnungen 145. Das Trennen der analytischen Hilfsmittel 134 bzw. der Verbindungselemente 146 kann beispielsweise durch mechanisches Stanzen, ausüben von Druck oder ähnliche Trennverfahren erfolgen, beispielsweise das oben beschriebene Brechen. Dabei können die einzelnen Lanzetten 136 beispielsweise an einem Vereinzelungswerkzeug fixiert werden, beispielsweise magnetisch und/oder mittels einer Vakuumfixierung oder einer ähnlichen Fixierungsvorrichtung, insbesondere solange, bis die Lanzetten 136 in ihren jeweiligen Kammern 122 liegen.

Aus dieser Darstellung geht hervor, dass ein Zweck der Aufnahmen 120 darin bestehen kann, die analytischen Hilfsmittel 134 nach dem Trennen von dem Halteelement 144 in ihrer räumlichen Ausrichtung zumindest teilweise zu fixieren. Entsprechend können die Aufnahmen 120 ausgestaltet sein, wobei diese nicht notwendigerweise, wie in den Figuren dargestellt, Vertiefungen umfassen müssen, so können diese Vertiefungen beispielsweise auch durch andere Elemente ersetzt werden und/oder grundsätzlich mit beliebig geringer Tiefe ausgestaltet werden. Die dargestellte Ausführungsform ist jedoch aufgrund der guten Fixierung bevorzugt, wobei vorzugsweise die Tiefe der Aufnahmen 120 mindestens gleich der Tiefe der Lanzetten 136 bzw. der analytischen Hilfsmittel 134 ausgestaltet wird.

Das Trennen der analytischen Hilfsmittel 134 kann vor, während oder nach dem Einfügen der analytischen Hilfsmittel 134 in die Aufnahmen 120 erfolgen. So kann beispielsweise ein Trennen nach dem Einlegen erfolgen und/oder bereits während die analytischen Hilfsmittel 134, noch über das Halteelement 144 miteinander verbunden, hängend über dem ersten Bauteil 112 positioniert sind. Das Trennen erfolgt für alle analytische Hilfsmittel 134 oder für mehrere analytische Hilfsmittel 134 gleichzeitig, so dass beispielsweise alle Lanzetten 136 auf einmal aus dem Halteelement 144 bzw. dem Ätzgitter herausgebrochen werden können, woraufhin diese in die darunter liegenden Aufnahmen 120 fallen können.
In einem weiteren Verfahrensschritt können die Aufnahmen 120 mit den darin aufgenommenen analytischen Hilfsmitteln 134 verschlossen werden. Dies kann grundsätzlich durch Aufbringen eines beliebigen zweiten Bauteils 148 erfolgen, welches beispielsweise auch in Form einer Folie ausgestaltet sein kann. In einem in Figur 4 dargestellten optionalen Fall ist das zweite Bauteil 148 jedoch beispielsweise als kreisringförmige Scheibe ausgestaltet, welche als Oberteil fungiert. Diese kreisringförmige Scheibe, wobei Figur 4 eine Ansicht schräg von unten (also eine Ansicht von den Kammern 122 her) dieses zweiten Bauteils 148 zeigt, kann beispielsweise ebenfalls aus Kunststoff hergestellt sein und ist vorzugsweise im Wesentlichen starr ausgestaltet.
Das zweite Bauteil 148 kann eine Mehrzahl von den Aufnahmen 120 korrespondierenden Elementen umfassen. Im dargestellten Ausführungsbeispiel umfasst das zweite Bauteil 148 eine Mehrzahl von Vertiefungen 150, welche zu den Aufnahmen 120 korrespondieren und welche ebenfalls radial ausgestaltet sind. Diese Vertiefungen 150 können beispielsweise, wie in Figur 4 angedeutet, wiederum Öffnungen 126 an der äußeren Umfangsseite 114 und/oder an der der Innenöffnung 116 zuweisenden Seite umfassen. Diese Öffnungen 126 können später Bestandteil der Probennahmeöffnungen 130 bzw. Aktoröffnungen 132 bilden.
Weiterhin umfasst das zweite Bauteil 148 eine Mehrzahl von ebenfalls zu den Aufnahmen 120 korrespondierend angeordneten Rippen 152. Diese Rippen können den analytischen Hilfsmitteln 134, beispielsweise den Lanzetten 136 bzw. Samplem, ggf. zusammen mit gebogenen Böden der Aufnahmen 120 des ersten Bauteils 112, eine elastische Biegung aufzwingen und diese auf diese Weise gegen Herausfallen sichern.
In Figur 5 ist das analytische Magazin 110 bzw. ein Halbfertigteil dieses analytischen Magazins 110 nach Aufbringen des zweiten Bauteils 148 auf das in Figur 3 gezeigte erste Bauteil 112 dargestellt. Gezeigt ist dabei eine perspektivische Darstellung schräg von oben, mit Blickrichtung auf das zweite Bauteil 148. Das zweite Bauteil 148 und das erste Bauteil 112 können beispielsweise miteinander verbunden werden, insbesondere durch ein Schweißverfahren, beispielsweise ein Laserschweißen. Zu diesem Zweck kann beispielsweise eines der Bauteile 112, 148 transparent für Laserstrahlung ausgestaltet sein, wohingegen das jeweils andere dieser Bauteile 112, 148 absorbierend für die Laserstrahlung ausgestaltet sein kann. Insbesondere können die beiden Bauteile 112, 148 also aus Materialien mit unterschiedlichen Absorptionseigenschaften hergestellt sein. Besonders bevorzugt ist die Verwendung eines oder mehrerer der oben dargestellten Kunststoffe. Dabei können für die beiden Bauteile 112, 148 grundsätzlich identische Grundwerkstoffe verwendet werden, beispielsweise gleiche Kunststoffe, welche sich jedoch in ihren Absorptionseigenschaften unterscheiden, beispielsweise durch Beimischung von Additiven wie beispielsweise Farbstoffen. Auf diese Weise kann insbesondere in einem Wellenlängenbereich zwischen 500 nm und 1200 nm, insbesondere zwischen 700 nm und 1100 nm, ein Unterschied in einer Absorption der Bauteile 112, 148 und/oder von Bestandteilen dieser Bauteile 112, 148 geschaffen werden, beispielsweise ein Unterschied in einer Absorption von mindestens 20%, vorzugsweise mindestens 50% oder sogar mindestens 80% oder mehr.

Aus den Figuren ist erkennbar, dass das zweite Bauteil 148 auf seiner in Figur 4 nicht erkennbaren Oberseite beispielsweise Transportelemente 118 bereitstellen kann. Weiterhin ist, was in Figur 4 nur schwer erkennbar ist, in Figur 5 deutlich erkennbar, dass das zweite Bauteil 148 eine Mehrzahl weiterer Öffnungen 126 in Form von Transferöffnungen 154 bereitstellt. Diese Transferöffnungen 154 können beispielsweise derart ausgestaltet sein, dass jeweils eine dieser Transferöffnungen 154 pro Kammer 122 vorgesehen ist. Dementsprechend sind die Transferöffnungen 154, welche im dargestellten Ausführungsbeispiel als runde Transferöffnungen 154 dargestellt sind, kreisförmig auf der Oberseite eines durch das erste Bauteil 112 und das zweite Bauteil 148 gebildeten Gehäuses 156 des Testelementmagazins 110 angeordnet. Auch andere Ausgestaltungen sind jedoch grundsätzlich möglich.

Die Transferöffnungen 154 in dem zweiten Bauteil 148 dienen später, im Einsatz des analytischen Magazins 110, beispielsweise in einem analytischen System, dazu, einen Transfer einer Probe von einer Lanzette 136 auf ein unten näher beschriebenes Testfeld 170 zu ermöglichen. Dementsprechend kann beispielsweise ein Aktor, beispielsweise ein Stößel, durch eine Transferöffnung 154 in eine Kammer 122 einzudringen, beispielsweise eine in einer Applikationsposition eines analytischen Systems befindliche Kammer 122, und die mit Probe beladene Lanzette 136 (bzw. den Microsampler) auf das Testfeld 170 zu drücken, so dass die Probe von der Lanzette 136 auf das Testfeld 170 übertragen wird. Die Transferöffnungen 154 können also auch als Aktoröffnungen 132 dienen und sind in den Figuren entsprechend als solche bezeichnet.

In einem weiteren Verfahrensschritt wird nun auf der Unterseite des Gehäuses 156 eine Testchemie 158 aufgebracht. Dies ist in Figur 7B angedeutet. Diese Testchemie 158 kann beispielsweise in Form eines Testchemiefeldes 160, insbesondere eines durchgehenden Testchemiefeldes 160, ausgestaltet sein, vorzugsweise in Form eines ringförmigen Testchemiefeldes 160. Die Testchemie 158 ist vorzugsweise derart ausgestaltet, dass diese zumindest weitgehend stabil ist gegenüber Umwelteinflüssen, insbesondere gegenüber Luftfeuchtigkeit. Auch eine Stabilität gegenüber herkömmlichen, für eine Sterilisation verwendeten ionisierenden Strahlungen ist vorteilhaft. Für mögliche Ausgestaltungen der Testchemie 158 kann auf die obige Beschreibung verwiesen werden.

Die Testchemie 158 kann beispielsweise auf einen Träger 164, beispielsweise einen ebenfalls kreisringförmigen Träger 164, aufgebracht werden, welcher vorzugsweise als durchgehende Träger 164 für alle Kammern 122, vorzugsweise als einstückiger Träger 164, ausgestaltet ist. Der Träger 164 und das Testchemiefeld 160 bilden zusammen in diesem Ausführungsbeispiel einen durchgehenden Chemiering 162. Beispielsweise kann der Träger 164 in Form einer selbstklebenden oder nicht-klebenden Folie und/oder eines Kunststoffträgers ausgestaltet sein. Auch andere Trägermaterialien sind grundsätzlich möglich. Dieser Träger 164 mit der Testchemie 158, welcher auf der in Figur 7B gezeigten Darstellung auf der Oberseite des Träges 164 angeordnet ist, wird im dargestellten Ausführungsbeispiel in die Ringnut 124 auf der Rückseite des ersten Bauteils 112 eingebracht. Durch die Testchemie 158 und den Träger 164 werden die Testelementöffnungen 128 vorzugsweise vollständig verschlossen, so dass der Träger 164 und/oder die Testchemie 158 gleichzeitig auch als Versiegelung 166 der Testelementöffnungen 128 wirken können. Alternativ oder zusätzlich kann jedoch auch eine zusätzliche Versiegelung 166 vorgesehen sein. Diese kann beispielsweise auf der Rückseite des Trägers 164 nach Aufbringen des Trägers 164 in die Ringnut 124, aufgebracht werden, beispielsweise durch ein Klebeverfahren und/oder Laminierverfahren.

Der Träger 164 bzw. das Testchemiefeld 160 sind vorzugsweise vollständig mit der Testchemie 158 bedeckt. In den Bereichen, in welchen das Testchemiefeld 160 die Testelementöffnungen 128 bedeckt, bilden die Bereiche 168 des Testchemiefeldes 160 jeweils Testfelder 170, welche dem Inneren der Kammern 122 zuweisen und welche somit ebenfalls Bestandteil der analytischen Hilfsmittel 134 bilden, bzw. Teil-Hilfsmittel.

Die Testchemie 158 liegt nun direkt unterhalb der Lanzetten 136 in den Kammern 122, und jede der Kammern 122 ist von der nächsten Kammer 122 getrennt. Lediglich die Probennahmeöffnungen 130, die Aktoröffnungen 132 und die Transferöffnungen 154 sind noch geöffnet. Wird nun eine Probennahmebewegung mittels der Lanzette 136 durchgeführt, so kann, beispielsweise über eine Kapillarwirkung und/oder eine Oberflächenwirkung der Lanzette 136, Körperflüssigkeit, insbesondere Blut, von dem Kapillarkanal 137 aufgenommen werden. Durch eine Rückbewegung der Lanzette 136 in die Kammer 122 gelangt die Körperflüssigkeit dann in die Nähe der Testchemie 158, so dass ein Probenübertrag von der Lanzette 136 auf die Testchemie 158 bzw. das entsprechende Testfeld 170 der gerade benutzten Kammer 122 erfolgen kann.

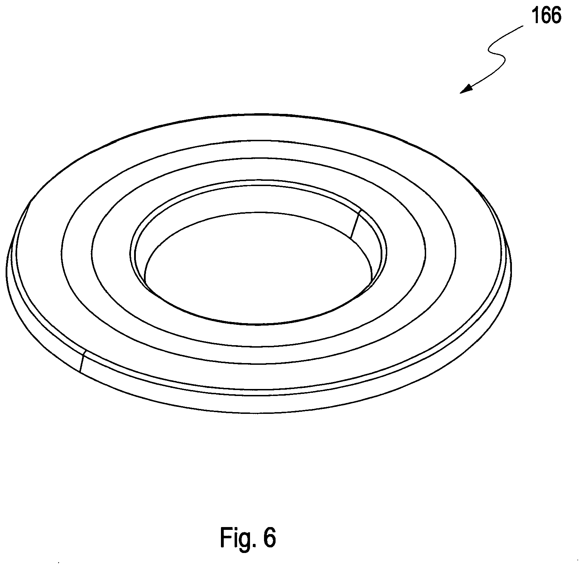

Um die momentan nicht verwendeten Kammern 122 gegenüber Umwelteinflüssen, insbesondere Feuchtigkeit, zu schützen, können die weiteren Öffnungen 126 in einem dem Verfahrensschritt in Figur 7B vorgelagerten, teilweise gleichzeitig erfolgenden oder nachgelagerten Verfahrensschritt versiegelt werden. So ist beispielsweise in Figur 6 eine Versiegelung 166 dargestellt, welche eingesetzt werden kann, um beispielsweise die Messöffnungen 154 und/oder die Aktoröffnungen 132 und/oder die Probennahmeöffnungen 130 gleichzeitig oder nacheinander zu versiegeln. Diese Versiegelung kann beispielsweise eine Ronde aus dünner Aluminiumfolie oder ähnlichen folienförmigen Elementen umfassen. Die Versiegelung 166, welche auch mehrstückig ausgebildet sein kann, kann beispielsweise mittels eines Tiefziehverfahrens vorgeformt werden. Die Versiegelung 166 kann beispielsweise formschlüssig und/oder stoffschlüssig und/oder kraftschlüssig, beispielsweise durch ein Verkleben und/oder Laminieren, mit dem Gehäuse 156 verbunden werden.

In Figur 7A ist das analytische Magazin 110 schließlich in versiegelter Form dargestellt. Dieses kann, wie oben dargestellt, beispielsweise in ein analytisches System eingelegt werden, in welchem das analytische Magazin 110 beispielsweise mittels eines entsprechenden Transportmechanismus um eine Rotationsachse gedreht werden kann, um beispielsweise jeweils eine der Kammern 122 in mindestens eine Applikationsposition, beispielsweise für eine Probennahmebewegung, zu bewegen. Weiterhin können weitere Positionen vorgesehen sein, beispielsweise Messpositionen, in welchen beispielsweise durch die Messöffnungen 154 eine Messung von Farbänderungen und/oder Änderungen anderer Eigenschaften der Testfelder 170 gemessen werden können.

In der Applikationsposition kann am Innenumfang, welcher der Innenöffnung 116 zuweist, ein Aktor, beispielsweise ein mindestens einen Betätigungsstößel umfassender Aktor, in die jeweils in der Applikationsposition befindliche Kammer 122 eingreifen, beispielsweise einstechen, wobei (beispielsweise gleichzeitig und/oder vorgelagert) die Versiegelung 166 der Aktoröffnung 132 der in der Applikationsposition befindlichen Kammer 122 geöffnet, beispielsweise durchstoßen werden kann. Durch die Versiegelung 166, beispielsweise die Folie, an der äußeren Umfangsseite 114, treten dann die Sampler in Form der Lanzetten 136 bei Betätigung aus.

Eine Messung von Eigenschaftsänderungen der Testfelder 170 kann beispielsweise von einer Außenseite des analytischen Magazins 110 her erfolgen, beispielsweise durch den Träger 164 der Testchemie 158 hindurch. Zu diesem Zweck kann der Träger 164 beispielsweise ganz oder teilweise transparent ausgestaltet sein, so dass beispielsweise in Figur 7B eine Messung von Farbänderungen von der Unterseite des Testelementmagazins 110 her erfolgen kann.

**Bezugszeichenliste**

| | | | |
|---|---|---|---|
| 110 | analytisches Magazin | 164 | Träger |
| 112 | erstes Bauteil | 166 | Versiegelung |
| 114 | äußere Umfangsseite | 168 | den Kammern zuweisender Bereich |
| 116 | Innenöffnung | | |
| 118 | Transportelement | 170 | Testfelder |
| 119 | Einkerbungen | | |
| 120 | Aufnahmen | | |
| 122 | Kammern | | |
| 124 | Ringnut | | |
| 126 | Öffnungen | | |
| 128 | Testelementöffnungen | | |
| 130 | Probennahmeöffnungen | | |
| 132 | Aktoröffnungen | | |
| 134 | analytische Hilfsmittel | | |
| 136 | Lanzetten | | |
| 137 | Kapillarkanal | | |
| 138 | Lanzettenende | | |
| 140 | Lanzettenkörper | | |
| 141 | Koppelelemente | | |
| 142 | Metallscheibe | | |
| 144 | Halteelement | | |
| 145 | Positionierungsöffnungen | | |
| 146 | Verbindungselemente | | |
| 147 | Verjüngungen | | |
| 148 | zweites Bauteil | | |
| 150 | Vertiefungen | | |
| 152 | Rippen | | |
| 154 | Transferöffnungen | | |
| 156 | Gehäuse | | |
| 158 | Testchemie | | |
| 160 | Testchemiefeld | | |
| 162 | Chemiering | | |

## Patentansprüche

1. Verfahren zur Herstellung eines analytischen Magazins (110), wobei das analytische Magazin (110) eingerichtet ist, um eine Mehrzahl von analytischen Hilfsmitteln (134) in einer Mehrzahl von Kammern (122) aufzunehmen, wobei das Verfahren folgende Schritte umfasst:
- Bereitstellen mindestens eines ersten Bauteils (112) des analytischen Magazins (110), wobei das erste Bauteil (112) eine Mehrzahl von Aufnahmen (120) umfasst,
- Bereitstellen einer Mehrzahl analytischer Hilfsmittel (134), wobei die analytischen Hilfsmittel (134) durch mindestens ein Halteelement (144) miteinander verbunden und vorzugsweise zueinander ausgerichtet sind,
- Einbringen der analytischen Hilfsmittel (134) in die Aufnahmen (120), wobei mehrere oder vorzugsweise alle Kammern (122) gleichzeitig bestückt werden; und
- Trennen der analytischen Hilfsmittel (134) von dem Halteelement (144), wobei das Trennen für alle oder für mehrere analytische Hilfsmittel (134) gleichzeitig erfolgt,
wobei die analytischen Hilfsmittel (134) zumindest teilweise einstückig mit dem Halteelement (144) hergestellt werden, insbesondere unter Verwendung mindestens eines Ätzprozesses, wobei die analytischen Hilfsmittel (134) Lanzetten (136) und/oder Microsampler umfassen, welche aus einem Grundwerkstoff des Halteelements (144) herausgearbeitet sind, so dass das Halteelement (144) und die analytischen Hilfsmittel (134) oder Teile derselben entstehen, wobei der Grundwerkstoff ein metallischer Grundwerkstoff ist.

2. Verfahren nach dem vorhergehenden Anspruch, wobei das Halteelement (144) mindestens eine Metallscheibe (142) aufweist.

3. Verfahren nach einem der vorhergehenden Ansprüche, wobei vor dem Trennen der analytischen Hilfsmittel (134) von dem Halteelement (144) zwischen den analytischen Hilfsmitteln (134) und dem Halteelement (144) mindestens eine Verbindung (146) mit mindestens einer Sollbruchstelle vorgesehen wird.

4. Verfahren nach einem der vorhergehenden Ansprüche, wobei alle analytische Hilfsmittel (134) derart auf einmal aus dem Halteelement (144) herausgebrochen werden, dass sie in die Aufnahmen (120), welche darunter angeordnet sind, fallen.

5. Verfahren nach einem der vorhergehenden Ansprüche, wobei die Kammern (122) und/oder die analytischen Hilfsmittel (134) derart ausgestaltet sind, dass die analytischen Hilfsmittel (134) für eine Probennahmebewegung ganz oder teilweise beweglich gelagert sind.

6. Verfahren nach einem der vorhergehenden Ansprüche, weiterhin umfassend folgenden Verfahrensschritt:
- Aufbringen mindestens eines zweiten Bauteils (148) des analytischen Magazins (110) auf das erste Bauteil (112).

7. Verfahren nach einem der vorhergehenden Ansprüche, weiterhin umfassend den folgenden Verfahrensschritt:
- Aufbringen mindestens einer Testchemie (158), wobei die Testchemie (158) eingerichtet ist, um bei Anwesenheit mindestens eines nachzuweisenden Analyten mindestens eine messbare Eigenschaft zu ändern, wobei das Aufbringen derart erfolgt, dass jeweils mindestens ein Bereich (168) der Testchemie (158) den Innenräumen der Kammern (122) zuweist und vom Inneren der Kammern (122) aus zugänglich ist.

8. Verfahren nach dem vorhergehenden Anspruch, wobei die mindestens eine Testchemie (158) in Form mindestens eines Testchemiefeldes (160), insbesondere eines durchgehenden Testchemiefeldes (160), aufgebracht wird, wobei das Testchemiefeld (160) auf einen durchgehenden Träger (164) aufgebracht ist, insbesondere einen Trägerring, wobei das Testchemiefeld (160) die Bereiche (168) der Testchemie (158) für mehrere Kammern (122) bereitstellt.

9. Verfahren nach dem vorhergehenden Anspruch, wobei das Testchemiefeld (160) durchgängig mit der Testchemie (158) bedeckt ist.

## Claims

1. Method for producing an analytical magazine (110), wherein the analytical magazine (110) is designed to receive a plurality of analytical aids (134) in a plurality of chambers (122), wherein the method comprises the following steps:
- providing at least one first component (112) of the analytical magazine (110), wherein the first component (112) comprises a plurality of receptacles (120),
- providing a plurality of analytical aids (134), wherein the analytical aids (134) are connected to one another and preferably oriented with respect to one another by means of at least one holding element (144),
- introducing the analytical aids (134) into the receptacles (120), wherein a plurality or preferably all of the chambers (122) are loaded simultaneously; and
- separating the analytical aids (134) from the holding element (144), wherein the separating takes place simultaneously for all or for a plurality of analytical aids (134),
wherein the analytical aids (134) are produced at least in part integrally with the holding element (144), in particular using at least one etching process, wherein the analytical aids (134) comprise lancets (136) and/or microsamplers which are worked from a basic material of the holding element (144), so that the holding element (144) and the analytical aids (134) or parts thereof are formed, wherein the basic material is a metallic basic material.

2. Method according to the preceding claim, wherein the holding element (144) has at least one metal disk (142).

3. Method according to any of the preceding claims, wherein, prior to separating the analytical aids (134) from the holding element (144), at least one connection (146) to at least one desired breaking location is provided between the analytical aids (134) and the holding element (144).

4. Method according to any of the preceding claims, wherein all analytical aids (134) are broken out of the holding element (144) at once so that they fall into the receptacles (120) which are arranged below.

5. Method according to any of the preceding claims, wherein the chambers (122) and/or the analytical aids (134) are configured in such a way that the analytical aids (134) are mounted such that they are movable wholly or in part for a sampling movement.

6. Method according to any of the preceding claims, furthermore comprising the following method step:
- applying at least one second component (148) of the analytical magazine (110) to the first component (112).

7. Method according to any of the preceding claims, furthermore comprising the following method step:
- applying at least one test chemical (158), wherein the test chemical (158) is designed to change at least one measurable property in the presence of at least one analyte to be detected, wherein the process of applying is effected in such a way that in each case at least one region (168) of the test chemical (158) faces the interiors of the chambers (122) and is accessible from inside the chambers (122).

8. Method according to the preceding claim, wherein the at least one test chemical (158) is applied in the form of at least one test chemical field (160), in particular a continuous test chemical field (160), wherein the test chemical field (160) is applied to a continuous carrier (164), in particular a carrier ring, wherein the test chemical field (160) provides the regions (168) of the test chemical (158) for a plurality of chambers (122).

9. Method according to the preceding claim, wherein the test chemical field (160) is covered with the test chemical (158) throughout.

## Revendications

1. Procédé destiné à la fabrication d'un chargeur pour analyses (110), selon lequel le chargeur pour analyses (110) est mis en place en vue de loger une pluralité de moyens auxiliaires (134) analytiques dans une pluralité de chambres (122), selon lequel ledit procédé comprend les phases suivantes :
- la préparation d'au moins un premier élément structurel (112) du chargeur pour analyses (110), selon lequel le premier élément structurel (112) comprend une pluralité de logements (120) ;
- la préparation d'une pluralité de moyens auxiliaires (134) analytiques, selon lequel les moyens auxiliaires (134) analytiques sont reliés les uns aux autres par l'intermédiaire d'au moins un élément de retenue (144) et sont de préférence alignés les uns par rapport aux autres ;
- la mise en place des moyens auxiliaires (134) analytiques dans les logements (120), selon lequel plusieurs ou, de préférence, toutes les chambres (122) sont alimentées en même temps ; et
- la séparation des moyens auxiliaires (134) analytiques, réalisée par l'élément de retenue (144), selon lequel la séparation est réalisée en même temps pour tous ou pour plusieurs moyens auxiliaires (134) analytiques ;
selon lequel les moyens auxiliaires (134) analytiques sont fabriqués d'une seule pièce, tout au moins en partie, avec l'élément de retenue (144), en particulier en utilisant au moins un processus de mordançage, selon lequel les moyens auxiliaires (134) analytiques comprennent des lancettes (136) et/ou des microsamplers, lesquels sont découpés à partir d'un matériau de base de l'élément de retenue (144), de telle sorte que l'élément de retenue (144) et les moyens auxiliaires (134) analytiques ou des parties de ceux-ci sont créés, selon lequel le matériau de base est un matériau de base métallique.

2. Procédé selon la revendication précédente, selon lequel l'élément de retenue (144) présente au moins un disque de métal (142).

3. Procédé selon l'une des revendications précédentes, selon lequel, avant la séparation des moyens auxiliaires (134) analytiques, au moins une jonction (146) est prévue avec au moins un point de rupture nominal par l'élément de retenue (144), entre les moyens auxiliaires (134) analytiques et l'élément de retenue (144).

4. Procédé selon l'une des revendications précédentes, selon lequel tous les moyens auxiliaires (134) analytiques sont fragmentés en une seule fois en dehors de l'élément de retenue (144), de telle sorte qu'ils viennent tomber dans les logements (120), lesquels sont disposés au-dessous.

5. Procédé selon l'une des revendications précédentes, selon lequel les chambres (122) et/ou les moyens auxiliaires (134) analytiques sont configurés de telle sorte que les moyens auxiliaires (134) analytiques sont positionnés de manière mobile, en totalité ou en partie, pour un déplacement du prélèvement des échantillons.

6. Procédé selon l'une des revendications précédentes, comprenant en outre la phase de procédé suivante :
- la mise en place d'au moins un deuxième élément structurel (148) du chargeur pour analyses (110) sur le premier élément structurel (112).

7. Procédé selon l'une des revendications précédentes, comprenant en outre la phase de procédé suivante :
- la mise en place d'au moins un processus de chimie de test (158), selon lequel le processus de chimie de test (158) est mis en place en vue de modifier au moins une propriété qui peut être mesurée en cas de la présence d'au moins d'un analyte que l'on cherche à détecter, selon lequel la mise en place est réalisée de telle sorte que respectivement au moins une zone (168) du processus de chimie de test (158) est attribuée aux compartiments intérieurs des chambres (122) et est accessible depuis l'intérieur des chambres (122).

8. Procédé selon la revendication précédente, selon lequel l'au moins un processus de chimie de test (158) est mis en place sous la forme d'au moins un champ de processus de chimie de test (160), en particulier d'un champ de processus de chimie de test continu (160), selon lequel le champ de processus de chimie de test (160) est mis en place sur un support (164) continu, en particulier une bague de support, selon lequel le champ de processus de chimie de test continu (160) met à disposition les zones (168) du processus de chimie de test (158) pour plusieurs chambres (122).

9. Procédé selon la revendication précédente, selon lequel le champ de processus de chimie de test (160) est couvert de manière continue avec le processus de chimie de test (158).
